# EUROPÄISCHE PATENTANMELDUNG

(11) **EP 1 932 513 A2**
(43) Veröffentlichungstag der Anmeldung: **18.06.2008**
(21) Anmeldenummer: 07023218.6
(22) Anmeldetag: 30.11.2007
(51) Int. Cl.: A61K 8/44, A61K 8/46, A61K 8/64, A61K 8/97, A61Q 19/00

(54) **Zusammensetzungen zur Verbesserung des optischen Erscheinungsbildes der Haut**

(30) Priorität: 11.12.2006 DE 102006058611
(71) Anmelder: Henkel AG & Co. KGaA, 40589 Düsseldorf (DE)
(72) Erfinder: Döring, Thomas, 41542 Dormagen (DE); Waldmann-Laue, Marianne, 40789 Monheim (DE); Janßen, Frank, 41470 Neuss (DE); Wadle, Armin, 40699 Erkrath (DE); Träger, Anemone, 40227 Düsseldorf (DE)

(57) **Zusammenfassung**

Gegenstand der Anmeldung sind topische kosmetische oder dermatologische Zusammensetzungen zur Behandlung extrinsisch gealterter oder lichtgeschädigter Haut, insbesondere zur Antifaltenbehandlung, zur Hemmung der zellulären Proteinoxidation, zur Steigerung bzw. Beibehaltung der Proteasomen-Aktivität unter UV-Einfluss, zur Steigerung der Lamininsynthese in der extrazellulären Matrix epidermaler Keratinozyten und zur Verbesserung des optischen Erscheinungsbildes der Haut.

## Beschreibung

Die vorliegende Anmeldung betrifft topische kosmetische oder dermatologische Zusammensetzungen zur Behandlung extrinsisch gealterter oder lichtgeschädigter Haut, insbesondere zur Antifaltenbehandlung.
Weiterhin betrifft die Erfindung topische kosmetische oder dermatologische Zusammensetzungen zur Hemmung der zellulären Proteinoxidation, zur Steigerung bzw. Beibehaltung der Proteasomen-Aktivität unter UV-Einfluss und zur Steigerung der Lamininsynthese in der extrazellulären Matrix epidermaler Keratinozyten.
Weiterhin betrifft die Erfindung topische kosmetische oder dermatologische Zusammensetzungen zur Verbesserung des optischen Erscheinungsbildes der Haut.

Die Haut ist täglich schädigender UV Strahlung ausgesetzt. Hierdurch kommt es zur Hautalterung und zur negativen Änderung des äußeren Erscheinungsbildes. Physiologisch bewirkt die UV-Strahlung, im besonderen UV-A Strahlung, unter anderem eine Oxidation der zellulären Proteine. Ein hieraus resultierendes Produkt sind sogenannte Proteincarbonyle.

Das Proteasom ist ein 700 kDa schwerer Proteinkomplex, der im Cytoplasma und im Zellkern Proteine zu Fragmenten abbaut. Das Proteasom in Eukaryoten besteht aus einer 20S- und zwei 19S-Untereinheiten, die ihrerseits wieder aus mehreren Proteinen zusammengesetzt sind. Den Proteasomen von Prokaryoten fehlen die 19S-Untereinheiten. Die Größe des Proteasoms ist 17x11 nm. Die 20S-Untereinheit ist ein hohler Zylinder und wirkt als multikatalytische Protease. An der Innenwand ist die proteolytische Aktivität lokalisiert. Die 19S-Komplexe sitzen als Deckel (cap) auf den beiden Öffnungen des 20S-Komplexes. Sie regulieren den Zugang zum 20S-Komplex und erkennen und entfalten zum Abbau bestimmte Proteine (nur entfaltete Proteine passen in den 20S-Komplex hinein). Zum Abbau bestimmte Proteine werden in einem mehrstufigen enzymatischen Prozess mit einer Polyubiquitin-Kette markiert und werden daran von den 19S-Komplexen erkannt. Ubiquitin ist ein kleines Molekül mit einem Molekulargewicht von 8,5 kDa. Die Polyubiquitin-Kette wird beim Abbau in ihre einzelnen Ubiquitin-Moleküle zerlegt, die dann wieder verwendet werden können. Der Proteinabbau ist für die Zelle lebensnotwendig. So werden metabolische Enzyme, Transkriptionsfaktoren oder auch den Zellzyklus regulierende Proteine wie Cycline, CDK-Inhibitoren oder Zellzyklusregulatoren degradiert. Ebenso werden fehlerhafte Proteine abgebaut. Auch die Peptide, die an den MHC I-Komplex gebunden auf der Oberfläche der Zelle dem Immunsystem präsentiert werden, werden im Proteasom prozessiert. Diese Funktionen machen das Proteasom zu einem zentralen Schalter innerhalb der Zelle. So ist das Proteasom auch dafür zuständig, die durch schädliche oxidative Prozesse entstandenen Proteincarbonyle abzubauen. Eine zu hohe Anzahl an Proteincarbonylen in der Zelle (beispielsweise durch starke oxidative Schädigung) kann allerdings zu einer Blockade des Proteasoms und damit zu Reduzierung der proteasomalen Aktivität führen.

### Stand der Technik

Im Stand der Technik sind Wirkstoffe bekannt, die als Schutzfilter gegen Strahlung des UV-A- und UV-B-Wellenlängenbereiches wirken. Bekannte Beispiele sind 3-(4-Methylbenzyliden)campher, 4-Aminobenzoesäurederivate, beispielsweise 4-(Dimethylamino)benzoesäure-2-ethylhexylester und 2-Cyano-3-phenyl-zimtsäure-2-ethylhexylester (Octocrylene).
Derartige Wirkstoffe werden topisch auf die Haut aufgetragen. Aufgrund ihrer Molekülstruktur sind Lichtschutzfilter in der Lage, Strahlung aus dem UV-A- und/oder UV-B-Bereich zu absorbieren und so die Dosis an schädlicher Strahlung wenigstens teilweise zu reduzieren.
Lichtschutzfilter sind allerdings nicht in der Lage, durch hochenergetische Strahlung hervorgerufene chemische Veränderungen an den Zellen der Haut zu reduzieren bzw. rückgängig zu machen. Außerdem können Lichtschutzfiltersubstanzen ein allergenes Potenzial aufweisen. Darüber hinaus weisen Lichtschutzfiltersubstanzen üblicherweise eher unangenehme kosmetische Eigenschaften, wie Klebrigkeit, auf und können die Austrocknung der Haut verstärken. Einige der handelsüblichen Lichtschutzfiltersubstanzen unterliegen auch bestimmten gesetzlichen Zulassungsbeschränkungen.

Weiterhin kennt der Stand der Technik zahlreiche Wirkstoffe, die den Schäden der extrinsischen Hautalterung, also insbesondere der Lichtalterung, entgegenwirken sollen. Hierzu zählen insbesondere Antioxidantien, aber auch DNA-Reparaturenzyme, wie Photolyase und Endonuclease T4N5.
Die Wirksamkeit der bekannten Substanzen ist allerdings eingeschränkt. Photolyase ist nur unter Lichteinfluss aktiv. Daher besteht weiterhin ein Bedürfnis nach alternativen Wirkstoffen, die gut verträglich sind und/oder eine höhere Wirksamkeit aufweisen als die bekannten Wirkstoffe.

Um die Zellen der Haut vor lichtinduzierten oxidativen Schäden zu bewahren, benötigt man also einerseits Wirkstoffe, die die Zahl der Proteincarbonyle senken. Andererseits benötigt man Wirkstoffe, die die proteasomale Aktivität fördern, um den Abbau entstandener, unerwünschter Proteincarbonyle zu begünstigen.

### Optisches Erscheinungsbild der Haut

Zur objektiven Charakterisierung des optischen Erscheinungsbildes der Haut stehen bereits zahlreiche instrumentelle Methoden zur Verfügung, beispielsweise die Colorimetrie oder Farbbildanalysen, vektorielle Reflektometrie und diverse optische Reliefmessungen. Hierbei bleiben aber der subjektive Faktor und die Erwartungen des Verbrauchers an ein gutes Produkt unberücksichtigt. Um auch subjektive Eindrücke über ein Hautbehandlungsprodukt zu standardisieren, werden, je nach Produkt und Behandlungsziel, diverse Kriterien zur sensorischen Charakterisierung identifiziert und mit Hilfe einer Gruppe von Personen, die die sensorische Charakterisierung sorgfältig anhand einer Vergleichsskala trainiert haben, sensorisch geprüft. Eine derartige Methode zur visuellen Beurteilung eines "strahlenden Teints/complexion radiance" ist die sogenannte C.L.B.T.^{™} -Sensormethode nach C. Musnier, P. Piquemal, P. Beau und J.C. Pittet, publiziert in Skin Research and Technology 2004, Vol. 10, Seiten 50 - 56. Nach diesem Verfahren werden sieben Parameter der Haut visuell durch ein trainiertes Panel erfasst: Coloring, das heißt die vier Hauptfarbtöne der Haut (rot-rosa, beige, olive und hell-rosa), Luminosity, Brightness und Transparency. Im Sinne der vorliegenden Anmeldung wird das Merkmal "complexion radiance" beziehungsweise der "strahlende Teint" nach der C.L.B.T.^{™} - Methode visuell in vivo geprüft.

### Aufgabenstellung

Eine Aufgabe der vorliegenden Anmeldung war es, alternative Wirkstoffe bereitzustellen, die den Schäden der extrinsischen Hautalterung, also insbesondere der Lichtalterung, entgegenwirken.
Eine weitere Aufgabe der vorliegenden Anmeldung war es, Wirkstoffe bereitzustellen, die den Schäden der extrinsischen Hautalterung, also insbesondere der Lichtalterung, entgegenwirken und die eine höhere Wirksamkeit aufweisen als die bekannten Substanzen.
Eine weitere Aufgabe der vorliegenden Anmeldung war es, Wirkstoffe bereitzustellen, die den Schäden der extrinsischen Hautalterung, also insbesondere der Lichtalterung, entgegenwirken und die eine hohe Hautverträglichkeit aufweisen.
Eine weitere Aufgabe der vorliegenden Anmeldung war es, für bekannte Wirkstoffe mit günstigen Effekten auf die Haut das neue Anwendungsgebiet, Schäden der extrinsischen Hautalterung, also insbesondere der Lichtalterung, entgegenzuwirken, zu identifizieren.
Eine weitere Aufgabe der vorliegenden Anmeldung war es, Wirkstoffe und Zusammensetzungen bereitzustellen, die das optische Erscheinungsbild der Haut, insbesondere die "complexion radiance", verbessern.

Überraschend wurde gefunden, dass ausgewählte Wirkstoffe in der Lage sind, die Bildung oxidierter Proteine zu verhindern bzw. die Anzahl bestimmter Oxidationsprodukte von Proteinen, nämlich der Proteincarbonyle, in der Zelle zu reduzieren. Weiterhin überraschend wurde gefunden, dass ausgewählte Wirkstoffe in der Lage sind, die Aktivität der Proteasomen unter UV-A-Einfluss zu steigern oder zumindest zu erhalten. Weiterhin überraschend wurde gefunden, dass ausgewählte Wirkstoffe in der Lage sind, einen besonders strahlenden Teint hervorzurufen bzw. die "complexion radiance" und damit das optische Erscheinungsbild der Haut zu verbessern.

Ein erster Gegenstand der vorliegenden Anmeldung ist die Verwendung von mindestens zwei verschiedenen Wirkstoffen, ausgewählt aus Glutamylamidoethylindol, Kombucha, Hypotaurin, Ascorbinsäure und deren Salzen, Ascorbinsäureestern und Ascorbinsäureethern, zur Herstellung einer kosmetischen oder pharmazeutischen Zusammensetzung zur Verbesserung der "complexion radiance".

Ein weiterer Gegenstand der vorliegenden Anmeldung ist die nicht-therapeutische kosmetische Verwendung von mindestens zwei verschiedenen Wirkstoffen, ausgewählt aus Glutamylamidoethylindol, Kombucha, Hypotaurin, Ascorbinsäure und deren Salzen, Ascorbinsäureestern und Ascorbinsäureethern, in einer kosmetischen Zusammensetzung zur Verbesserung der "complexion radiance".

Ein weiterer Gegenstand der vorliegenden Anmeldung ist die Verwendung von mindestens einem Wirkstoff, ausgewählt aus Glutamylamidoethylindol, Kombucha, Hypotaurin, Ascorbinsäure und deren Salzen, Ascorbinsäureestern, Ascorbinsäureethern und Mischungen dieser Wirkstoffe, zur Herstellung einer kosmetischen oder pharmazeutischen Zusammensetzung zur Reduzierung der zellulären Proteinoxidation in Keratinozyten.

Ein weiterer Gegenstand der vorliegenden Anmeldung ist die nicht-therapeutische kosmetische Verwendung von mindestens einem Wirkstoff, ausgewählt aus Glutamylamidoethylindol, Kombucha, Hypotaurin, Ascorbinsäure und deren Salzen, Ascorbinsäureestern, Ascorbinsäureethern und Mischungen dieser Wirkstoffe, in einer kosmetischen Zusammensetzung zur Reduzierung der zellulären Proteinoxidation in Keratinozyten.

Ein weiterer Gegenstand der vorliegenden Anmeldung ist die Verwendung von mindestens einem Wirkstoff, ausgewählt aus Glutamylamidoethylindol, Kombucha, Hypotaurin, Ascorbinsäure und deren Salzen, Ascorbinsäureestern, Ascorbinsäureethern und Mischungen dieser Wirkstoffe, zur Herstellung einer kosmetischen oder pharmazeutischen Zusammensetzung zur Steigerung der Proteasomen-Aktivität in Keratinozyten bzw. zur Beibehaltung der Proteasomen-Aktivität in Keratinozyten unter UV-Einfluss.

Ein weiterer Gegenstand der vorliegenden Anmeldung ist die nicht-therapeutische kosmetische Verwendung von mindestens einem Wirkstoff, ausgewählt aus Glutamylamidoethylindol, Kombucha, Hypotaurin, Ascorbinsäure und deren Salzen, Ascorbinsäureestern, Ascorbinsäureethern und Mischungen dieser Wirkstoffe, in einer kosmetischen Zusammensetzung zur Steigerung der Proteasomen-Aktivität in Keratinozyten bzw. zur Beibehaltung der Proteasomen-Aktivität in Keratinozyten unter UV-Einfluss.

Ein weiterer Gegenstand der vorliegenden Anmeldung ist die Verwendung von Kombucha zur Herstellung einer kosmetischen oder pharmazeutischen Zusammensetzung zur Steigerung der Lamininsynthese in der extrazellulären Matrix epidermaler Keratinozyten.

Ein weiterer Gegenstand der vorliegenden Anmeldung ist die nicht-therapeutische kosmetische Verwendung von Kombucha in einer kosmetischen Zusammensetzung zur Steigerung der Lamininsynthese in der extrazellulären Matrix epidermaler Keratinozyten.

Erfindungsgemäß besonders bevorzugt ist die Verwendung einer Wirkstoffkombination von Glutamylamidoethylindol und Kombucha zur Verbesserung der "complexion radiance", zur Reduzierung der zellulären Proteinoxidation in Keratinozyten oder zur Steigerung bzw. Beibehaltung der Proteasomen-Aktivität in Keratinozyten unter UV-Einfluss.

Der Wirkstoff Glutamylamidoethylindol weist die untenstehende Strukturformel (I) auf und ist z. B. als wässrige Lösung unter der Handelsbezeichnung Glistin von der Firma Exsymol erhältlich.

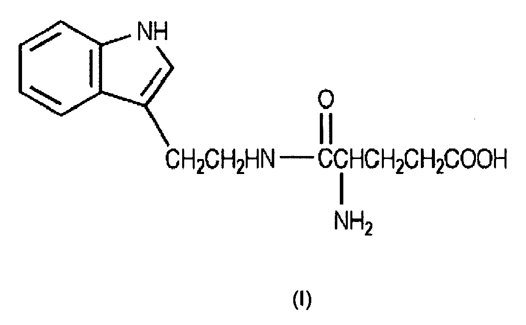

Bei Kombucha handelt es sich um ein aus dem ostasiatischen Raum stammendes, etwa 0,5 Vol.-% Alkohol enthaltendes, angenehm säuerliches, leicht süßes, moussierendes Gärungsgetränk auf Basis von gezuckertem Tee (Schwarzer Tee, Grüner Tee, Kräutertee oder Früchtetee), das ohne Zusatz von Konservierungsstoffen, Farbstoffen und Aromastoffen hergestellt wird.
Beim "Kombucha-Teepilz" handelt es sich tatsächlich nicht um einen Pilz, sondern um eine Symbiose von Essigsäurebakterien (meistens *Acetobacter xylinum,* aber auch *Acetobacter gluconicum*) mit verschiedenen Hefen (*Saccharomyces* sp., *Torula* sp., *Pichia fermentans*); fakultativ können Milchsäurebakterien vorhanden sein. Aus Gründen der Produktionssicherheit und Produktionsstabilität wird industriell gefertigter Kombucha oft wärmebehandelt.

Unter "Tee" werden erfindungsgemäß wässrige Aufgüsse von Blättern, Knospen und zarten Stielen von Thea sinensis (Camellia sinensis) und Thea assamica, aber auch von diversen anderen Pflanzen verstanden, beispielsweise Minze, Linde (Lindenblüten), Malve, Rotbusch, Kamille usw.

Bevorzugte Kombucha ist aus Tee von fermentierten (Schwarztee, Oolong-Tee, Gelber Tee, Pu-Erh-Tee) oder unfermentierten (Grüntee) Blättern von Thea sinensis (Camellia sinensis) oder Thea assamica gewonnen. Weitere bevorzugte Kombucha ist durch Fermentation von (meist mit Saccharose) gezuckertem Tee mit den symbiotischen Mikroorganismen Saccharomyces und Acetobacter xylinum gewonnen. Ein erfindungsgemäß besonders bevorzugtes Kombucha-Produkt ist ein Produkt, das durch Fermentation von mit Saccharose gezuckertem Schwarztee mit den zwei symbiotischen Mikroorganismen Saccharomyces und Acetobacter xylinum gewonnen wurde und beispielsweise unter der INCl-Bezeichnung "Saccharomyces/Xylinum/Black Tea Ferment, Glycerin, Hydroxyethylcellulose" mit der Handelsbezeichnung "Kombuchka" als Glycerin-haltige verdickte Zubereitung von der Firma Sederma erhältlich ist.

Besonders bevorzugt ist die nicht-therapeutische kosmetische Verwendung einer Kombination der Wirkstoffe Glutamylamidoethylindol und Kombucha in einer kosmetischen Zusammensetzung zur Verbesserung der "complexion radiance".

Ebenfalls besonders bevorzugt ist die Verwendung einer Kombination der Wirkstoffe Glutamylamidoethylindol und Kombucha zur Herstellung einer kosmetischen oder pharmazeutischen Zusammensetzung zur Verbesserung der "complexion radiance".

Ebenfalls besonders bevorzugt ist die Verwendung von einer Kombination der Wirkstoffe Glutamylamidoethylindol und Kombucha zur Herstellung einer kosmetischen oder pharmazeutischen Zusammensetzung zur Reduzierung der zellulären Proteinoxidation in Keratinozyten.

Ebenfalls besonders bevorzugt ist die Verwendung von einer Kombination der Wirkstoffe Glutamylamidoethylindol und Kombucha zur Herstellung einer kosmetischen oder pharmazeutischen Zusammensetzung zur Steigerung der Proteasomen-Aktivität in Keratinozyten bzw. zur Beibehaltung der Proteasomen-Aktivität in Keratinozyten unter UV-Einfluss.

Erfindungsgemäß besonders bevorzugt verwendete Zusammensetzungen mit einem Gehalt an Glutamylamidoethylindol und Kombucha sind dadurch gekennzeichnet, dass 0,0001 - 0,1 Gew.-%, bevorzugt 0,001 - 0,05 Gew.-%, besonders bevorzugt 0,001 - 0,02 Gew.-%, jeweils bezogen auf das Gesamtgewicht der Zusammensetzung, an Glutamylamidoethylindol enthalten ist.

Weitere erfindungsgemäß besonders bevorzugt verwendete Zusammensetzungen mit einem Gehalt an Glutamylamidoethylindol und Kombucha sind dadurch gekennzeichnet, dass 0,0001 - 10 Gew.-%, bevorzugt 0,01 - 5 Gew.-%, besonders bevorzugt 0,1 - 3 Gew.-%, jeweils bezogen auf das Gesamtgewicht der Zusammensetzung, an Kombucha enthalten ist.

Weitere erfindungsgemäß besonders bevorzugt verwendete Zusammensetzungen mit einem Gehalt an Glutamylamidoethylindol und Kombucha sind dadurch gekennzeichnet, dass 0,0001 - 0,1 Gew.-%, bevorzugt 0,001 - 0,05 Gew.-%, besonders bevorzugt 0,001 - 0,02 Gew.-%, jeweils bezogen auf das Gesamtgewicht der Zusammensetzung, an Glutamylamidoethylindol sowie 0,0001 - 10 Gew.-%, bevorzugt 0,01 - 5 Gew.-%, besonders bevorzugt 0,1 - 3 Gew.-%, jeweils bezogen auf das Gesamtgewicht der Zusammensetzung, an Kombucha enthalten ist.

Weitere erfindungsgemäß besonders bevorzugt verwendete Zusammensetzungen mit einem Gehalt an Glutamylamidoethylindol und Kombucha sind dadurch gekennzeichnet, dass 0,0001-0,1 Gew.-%, bevorzugt 0,001 - 0,05 Gew.-%, besonders bevorzugt 0,001 - 0,02 Gew.-%, jeweils bezogen auf das Gesamtgewicht der Zusammensetzung, an Glutamylamidoethylindol sowie 0,0001 - 10 Gew.-%, bevorzugt 0,01 - 5 Gew.-%, besonders bevorzugt 0,1 - 3 Gew.-%, jeweils bezogen auf das Gesamtgewicht der Zusammensetzung, an Kombucha enthalten ist.

Weitere erfindungsgemäß besonders bevorzugt verwendete Zusammensetzungen mit einem Gehalt an Glutamylamidoethylindol und Kombucha sind dadurch gekennzeichnet, dass das Gewichtsverhältnis von Kombucha zu Glutamylamidoethylindol, jeweils bezogen auf den Aktivsubstanzgehalt, im Bereich von 0,1 bis 100, bevorzugt im Bereich von 1 bis 20 und besonders bevorzugt im Bereich von 2 bis 10 liegt.

Weitere erfindungsgemäß bevorzugt verwendete topische Zusammensetzungen sind dadurch gekennzeichnet, dass mindestens ein Wirkstoff, ausgewählt aus Glutamylamidoethylindol, Kombucha, Hypotaurin, Ascorbinsäure und deren Salzen, Ascorbinsäureestern, Ascorbinsäureethern und Mischungen dieser Wirkstoffe, in einer Menge von 0,001 - 10 Gew.-%, bevorzugt 0,01 - 1 Gew.-%, besonders bevorzugt 0,02 - 0,5 Gew.-%, und außerordentlich bevorzugt 0,05 - 0,3 Gew.-%, jeweils bezogen auf das Gewicht der gesamten Zusammensetzung, enthalten ist.

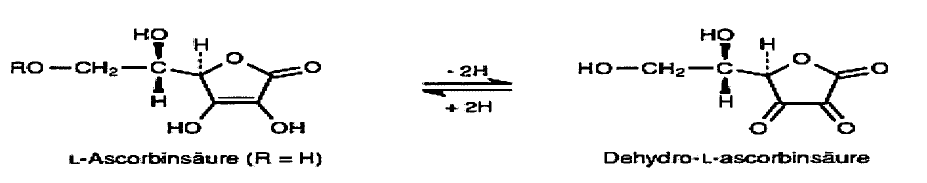

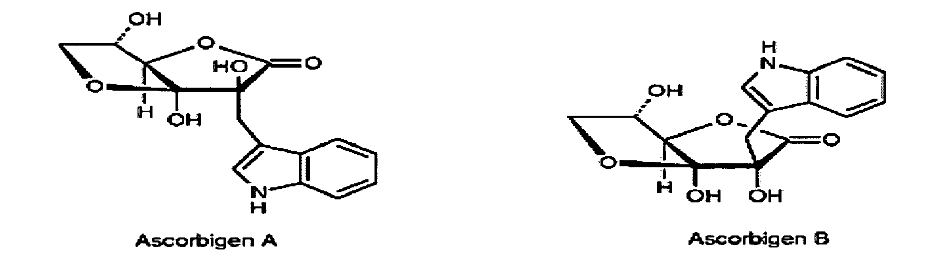

Erfindungsgemäß bevorzugt bzw. bevorzugt verwendet sind die Ascorbinsäure selbst, insbesondere L-Ascorbinsäure, weiterhin Dehydroascorbinsäure. Erfindungsgemäß bevorzugt verwendete Ascorbinsäurederivate sind insbesondere die Salze der Ascorbinsäure, d.h. Hydroascorbate bzw. Ascorbate, wobei unter diesen die Alkali- und Erdalkalisalze bevorzugt sind. Besonders bevorzugte Salze der Ascorbinsäure sind Natriumhydroascorbat, Natriumascorbat, Kaliumhydroascorbat, Kaliumascorbat, Ammoniumascorbat, Ammoniumhydroascorbat, Calciumascorbat, Calciumhydroascorbat, Magnesiumascorbat und Magnesiumhydroascorbat.

Weitere erfindungsgemäß bevorzugte bzw. bevorzugt verwendete Ascorbinsäurederivate sind ausgewählt aus den Estern der Ascorbinsäure mit mindestens einer organischen und/oder anorganischen Säure (R ≠ H in der vorstehend aufgeführten Formel). Unter den Estern der Ascorbinsäure sind insbesondere Ascorbylmethanoat, Ascorbylethanoat, Ascorbyl-n-propanoat, Ascorbyl-iso-propanoat, Ascorbyl-n-butanoat, usw. bevorzugt. Mit besonderem Vorzug werden erfindungsgemäß die Monoester, Diester, Triester und Tetraester von Ascorbinsäure mit Fettsäuren verwendet, insbesondere Ascorbyldipalmitat, Ascorbylisostearat, Ascorbyllinoleat, Ascorbyloleat, Ascorbyllinoleinat, Ascorbylpalmitat (6-Palmitoyl-L-ascorbinsäure), Ascorbylstearat, und Ascorbyltetraisopalmitat.

Auch anorganische Säuren können mit Ascorbinsäure verestert werden. Unter den Estern der Ascorbinsäure mit anorganischen Säuren ist insbesondere das Ascorbylphosphat bevorzugt, insbesondere in Form des Natriumsalzes, Natriumascorbylphosphat, oder des Magnesiumsalzes, Magnesiumascorbylphosphat.

Weitere erfindungsgemäß bevorzugte bzw. bevorzugt verwendete Ascorbinsäurederivate sind ausgewählt aus den Ethern der Ascorbinsäure mit mindestens einer organischen Polyhydroxyverbindung, insbesondere mit mindestens einem (glycosidisch gebundenen) Saccharid, insbesondere Ascorbylglucosid.

Weitere erfindungsgemäß bevorzugt verwendete Ascorbinsäurederivate sind ausgewählt aus Aminopropyl Ascorbyl Phosphate, Ascorbyl Inositol Niacinate, Ascorbyl Methylsilanol Pectinate, Ascorbyl Niacinamide, Ascorbyl Pentapeptide, Ascorbyl Pentapeptide-2, Ascorbyl Phosphate Succinoyl Pentapeptide-12, Ascorbyl Tocopheryl Maleate, Disodium Ascorbyl Sulfate, Disodium Isostearyl Ascorbyl Phosphate, Magnesium Ascorbylborate, Magnesium Ascorbyl Phosphate, Magnesium L-ascorbyl-2-phosphate, Potassium Ascorbylborate, Potassium Ascorbyl Tocopheryl Phosphate, Sodium Ascorbyl/Cholesteryl Phosphate, Sodium Ascorbyl Phosphate, Trisodium Ascorbyl Isopalmitate Phosphate und Trisodium Ascorbyl Palmitate Phosphate.

Erfindungsgemäß besonders bevorzugt verwendete kosmetische oder pharmazeutische Zubereitungen sind dadurch gekennzeichnet, dass sie, bezogen auf ihr Gewicht, insgesamt 0,05 bis 3,0 Gew.-%, vorzugsweise 0,07 bis 2,0 Gew.-% und insbesondere 0,1 bis 1,0 Gew.-% Ascorbinsäure und/oder Ascorbinsäuresalze und/oder Ascorbinsäurederivate enthalten.

Weiterhin wurde überraschend festgestellt, dass die erfindungsgemäß verwendeten Zusammensetzungen in ihrer Wirkung weiter in einem Ausmaß gesteigert werden können, die für den Fachmann nicht vorherzusehen war, indem mindestens ein kosmetischer Wirkstoff zugefügt wird, der ausgewählt ist aus Monomeren und Oligomeren von Aminosäuren, N-C₂-C₂₄-Acylaminosäuren, den Estern und/oder den physiologisch verträglichen Metallsalzen dieser Substanzen, DNA-Reparaturenzymen, DNA- oder RNA-Oligonucleotiden, Vitaminen, Provitaminen und Vitaminvorstufen der Gruppen A, B, E, H und K und den Estern der vorgenannten Substanzen, α-Hydroxycarbonsäuren, α-Ketocarbonsäuren, β-Hydroxycarbonsäuren und deren Ester-, Lacton- und/oder Salzform, Flavonoiden und Flavonoid-reichen Pflanzenextrakten, Ubichinon und Ubichinol sowie deren Derivaten, Silymarin, Ectoin, anorganischen und organischen UV-Filtersubstanzen, selbstbräunenden, hautberuhigenden, feuchtigkeitsspendenden und sebumregulierenden Wirkstoffen sowie Mischungen dieser Wirkstoffe.

Erfindungsgemäß besonders bevorzugt verwendete kosmetische oder dermatologische Zusammensetzungen zur topischen Behandlung der Haut sind dadurch gekennzeichnet, dass sie weiterhin mindestens einen kosmetischen Wirkstoff enthalten, der ausgewählt ist aus:
- Monomeren und Oligomeren von Aminosäuren, N-C₂-C₂₄-Acylaminosäuren, den Estern und/oder den physiologisch verträglichen Metallsalzen dieser Substanzen,
- DNA-Reparaturenzymen,
- DNA- oder RNA-Oligonucleotiden,
- Vitaminen, Provitaminen und Vitaminvorstufen der Gruppen A, B, E, H und K und den Estern der vorgenannten Substanzen,
- α-Hydroxycarbonsäuren, α-Ketocarbonsäuren, β-Hydroxycarbonsäuren und deren Ester-, Lacton- und/oder Salzform,
- Flavonoiden und Flavonoid-reichen Pflanzenextrakten,
- Ubichinon und Ubichinol sowie deren Derivaten,
- Silymarin,
- Ectoin,
- anorganischen und organischen UV-Filtersubstanzen,
- selbstbräunenden,
- hautberuhigenden,
- feuchtigkeitsspendenden und
- sebumregulierenden Wirkstoffen sowie
- Mischungen dieser Wirkstoffe.
Die Monomere der Aminosäuren und/oder der N-C₂-C₂₄-Acylaminosäuren sind bevorzugt ausgewählt aus Alanin, Arginin, Asparagin, Asparaginsäure, Canavanin, Citrullin, Cystein, Cystin, Desmosin, Glutamin, Glutaminsäure, Glycin, Histidin, Homophenylalanin, Hydroxylysin, Isodesmosin, Isoleucin, Leucin, Lysin, Methionin, Methylnorleucin, Ornithin, Phenylalanin, Prolin, Pyroglutaminsäure, Sarcosin, Serin, Taurin, Threonin, Thyroxin, Tryptophan, Tyrosin, Valin, Zinkpyroglutamat, Natriumoctanoylglutamat, Natriumdecanoylglutamat, Natriumlauroylglutamat, Natriummyristoylglutamat, Natriumcetoylglutamat und Natriumstearoylglutamat. Besonders bevorzugt sind Lysin, Serin, Taurin, Zink- und Natriumpyroglutamat und Natriumlauroylglutamat.
Der C₂ - C₂₄-Acylrest, mit dem die genannten Aminosäuren an der Aminogruppe derivatisiert sind, ist ausgewählt aus einem Acetyl-, Propanoyl-, Butanoyl-, Pentanoyl-, Hexanoyl-, Heptanoyl-, Octanoyl-, Nonanoyl-, Decanoyl-, Undecanoyl-, Lauroyl-, Tridecanoyl-, Myristoyl-, Pentadecanoyl-, Cetoyl-, Palmitoyl-, Stearoyl-, Elaidoyl-, Arachidoyl- oder Behenoyl-Rest. Mischungen von C₈-C₁₈-Acylresten werden auch als Cocoyl-Rest bezeichnet und sind ebenfalls bevorzugte Substituenten.

Die physiologisch verträglichen Salze der erfindungsgemäß bevorzugten Wirkstoffe, die Säuregruppen enthalten und Salze bilden können, sind ausgewählt aus den Ammonium-, Alkalimetall-, Magnesium-, Calcium-, Aluminium-, Zink- und Mangan-Salzen. Bevorzugt sind die Natrium-, Kalium-, Magnesium-, Aluminium-, Zink- und Mangan-Salze.

Die Oligomere der Aminosäuren und/oder der N-C₂-C₂₄-Acylaminosäuren sind bevorzugt ausgewählt aus Di-, Tri-, Tetra-, Penta-, Hexa- oder Pentadecapeptiden, die N-acyliert und/oder verestert sein können. Erfindungsgemäß bevorzugte, gegebenenfalls N-acylierte und/oder veresterte Dipeptide sind Tyr-Arg (Dipeptide-1), Val-Trp (Dipeptide-2), Asn-Phe, Asp-Phe, N-Palmitoyl-β-Ala-His, N-Acetyl-Tyr-Arghexyldecylester (z. B. Calmosensine von Sederma), Carnosin (β-Ala-His) und N-Palmitoyl-Pro-Arg. Erfindungsgemäß besonders bevorzugte, gegebenenfalls N-acylierte und/oder veresterte Tripeptide sind Lys-Pro-Val, Tyr-Tyr-Val, Tyr-Val-Tyr, Val-Tyr-Val (Tripeptide-2), Tripeptide-4 (z. B. ATPeptide, zu beziehen über IMPAG), His-Ala-Orn und N-Acetyl-Arg-Lys-Arg-NH₂. Erfindungsgemäß bevorzugte, gegebenenfalls N-acylierte und/oder veresterte Pentapeptide N-Palmitoyl-Tyr-Gly-Gly-Phe-Met, Val-Val-Arg-Pro-Pro, N-Palmitoyl-Tyr-Gly-Gly-Phe-Leu, Gly-Pro-Phe-Pro-Leu und N-Benzyloxycarbonyl-Gly-Pro-Phe-Pro-Leu (die beiden letztgenannten stellen Serinproteinase-Inhibitoren zur Inhibition der Desquamation dar). Erfindungsgemäß bevorzugte, gegebenenfalls N-acylierte und/oder veresterte Hexapeptide sind Ala-Arg-His-Leu-Phe-Trp (Hexapeptide-1), Acetyl Hexapeptide-1 (z. B. Modulene von Vincience), Acetyl Glutamyl Hexapeptide-1 (z. B. SNAP-7 von Centerchem), Hexapeptide-2 (z. B. Melanostatine-DM von Vincience), Val-Val-Arg-Pro-Pro-Pro, Ala-Arg-His-Methylnorleucin-Homophenylalanin-Trp (Hexapeptide-7) und Hexapeptide-11 (z. B. Peptamide-6 von Arch Personal Care). Ein erfindungsgemäß besonders bevorzugtes Pentadecapeptid ist z. B. der Rohstoff Vinci 01 von Vincience (Pentadecapeptide-1).

Erfindungsgemäß bevorzugte DNA-Reparaturenzyme sind Photolyase und T4 Endonuclease V, letztere im Weiteren mit "T4N5" abgekürzt. Diese beiden Enzyme sind im Stand der Technik bereits als sogenannte DNA-Reparatur-Enzyme bekannt. Unter DNA-Reparatur ist definitionsgemäß die Spaltung bzw. Entfernung von UV-induzierten Pyrimidindimeren aus der DNA zu verstehen.
Photolyase ist die Kurzbezeichnung für Desoxyribodipyrimidin-Photolyase bzw. DNA-Photolyase, ein Enzym mit der Klassifizierungsnummer EC 4.1.99.3. Eine besonders effiziente Photolyase stammt aus *Anacystis nidulans,* einem phototrophen marinen Mikroorganismus. Die Photolyase aus *A. nidulans* wird in technisch relevanten Mengen mittlerweile aus E. coli gewonnen. Photolyase ist zur Aktivierung auf Licht angewiesen.
Das Enzym T4 Endonuclease V wird vom denV-Gen der Bakteriophage T4 produziert und gehört zu den Phosphodiesterasen, die die Nucleinsäuren an der (5'-3')-Bindung hydrolytisch spalten. T4N5 ist auch ohne Lichteinfluss aktiv.

Erfindungsgemäß besonders bevorzugt ist der Einsatz von liposomenverkapselten DNA-Reparaturenzymen. Liposomenverkapselte Photolyase ist im Handel z. B. unter der Produktbezeichnung Photosomes^{™} (INCI-Bezeichnung: Water, Lecithine, Plankton Extract) mit einer Proteinkonzentration von 0,25 - 2 mg/ml, liposomenverkapselte T4N5 z. B. unter der Bezeichnung Ultrasomes^{™} (INCI-Bezeichnung: Water, Lecithine, Micrococcus Luteus Extract) mit einer Proteinkonzentration von 2 - 5 mg/ml (gemessen mit dem Bradford-Assay) von AGI Dermatics, USA, erhältlich.
Erfindungsgemäß besonders bevorzugt verwendete kosmetische oder dermatologische Zusammensetzungen zur topischen Behandlung der Haut sind dadurch gekennzeichnet, dass sie weiterhin mindestens einen kosmetischen Wirkstoff, der ausgewählt ist aus Photosomes^{™} und/ oder Ultrasomes^{™}, in Gesamtmengen von 0,1 - 10 Gew.-%, bevorzugt 0,5 - 5,0 Gew.-% und besonders bevorzugt 1,0-4,0 Gew.-%, bezogen auf das gesamte Mittel, enthalten.
Erfindungsgemäß besonders bevorzugt verwendete kosmetische oder dermatologische Zusammensetzungen zur topischen Behandlung der Haut sind dadurch gekennzeichnet, dass sie weiterhin mindestens einen kosmetischen Wirkstoff, der ausgewählt ist aus den Monomeren und Oligomeren von Aminosäuren, N-C₂-C₂₄-Acylaminosäuren und/oder den Estern und/oder den physiologisch verträglichen Metallsalzen dieser Substanzen, in Gesamtmengen von 0,00001 - 10 Gew.-%, bevorzugt 0,001 - 5 Gew.-% und besonders bevorzugt 0,005 - 3 Gew.-%, jeweils bezogen auf die gesamte Zusammensetzung, enthalten.

In einer weiteren bevorzugten Ausführungsform der Erfindung liegen die Monomeren und Oligomeren von Aminosäuren, N-C₂-C₂₄-Acylaminosäuren, den Estern und/oder den physiologisch verträglichen Metallsalzen dieser Substanzen in geträgerter Form vor, insbesondere aufgetragen auf feinteiligen, pulverförmigen Substraten wie Kieselgel, insbesondere Aerosil-Typen, weiterhin Talkum, Microsponges, modifizierten Stärken und Stärkederivaten, kristalliner Cellulose, Cellulosepulvern, Lactogiobulinderivaten, Polymerpartikeln aus Nylon, Polyolefinen, Polycarbonaten, Polyurethanen, Polyacrylaten, (Meth)acrylat- oder (Meth)acrylat-Vinyliden-Copolymeren, die vernetzt sein können, Polyestern, Polyamiden, Polystyrolen, Teflon und Siliconen. Ein besonders bevorzugter Rohstoff dieser Art sind die Vegetal Filling Spheres von Coletica.

Erfindungsgemäß besonders bevorzugt verwendete kosmetische oder dermatologische Zusammensetzungen zur topischen Behandlung der Haut sind dadurch gekennzeichnet, dass sie weiterhin mindestens einen kosmetischen Wirkstoff enthalten, der ausgewählt ist aus DNA-Oligonucleotiden und RNA-Oligonucleotiden.
Erfindungsgemäß werden unter einem Oligonucleotid Polymerisate aus 2 bis 20, bevorzugt 2 bis 10 Mononucleotiden verstanden, die ebenso wie bei Polynucleotiden und Nucleinsäuren durch Phosphorsäurediester-Brücken verknüpft sind. Die Nucleotide bestehen aus Nucleobasen (meist Pyrimidin- oder Purin-Derivaten), Pentosen (meist D-Ribofuranose oder 2-Desoxy-D-ribofuranose in β-N-glycosidischer Bindung an die Nucleobase) und Phosphorsäure. Die Mononucleotide sind zum Beispiel Adenosinphosphate, Cytidinphosphate, Guanosinphosphate, Uridinphosphate und Thymidinphosphate, insbesondere CMP (Cytidin-5'-monophosphat), UDP (Uridin-5'-diphosphat), ATP (Adenosin-5'-triphosphat) und GTP (Guanosin-5'-triphosphat).
Ein erfindungsgemäß besonders bevorzugtes Oligonucleotid ist das Thymidin-Dinucleotid.
In den besonders bevorzugt verwendeten erfindungsgemäßen Zusammensetzungen sind die DNA-Oligonucleotide oder RNA-Oligonucleotide in Mengen von 0,00001 - 5 Gew.-%, bevorzugt 0,0001 - 1,0 Gew.-% und besonders bevorzugt 0,0005 - 0,5 Gew.-%, bezogen auf die gesamte Zusammensetzung, enthalten.

Erfindungsgemäß besonders bevorzugt verwendete kosmetische oder dermatologische Zusammensetzungen zur topischen Behandlung der Haut sind dadurch gekennzeichnet, dass sie weiterhin mindestens einen kosmetischen Wirkstoff enthalten, der ausgewählt ist aus mindestens einem Vitamin, Provitamin oder einer als Vitaminvorstufe bezeichnete Verbindung aus den Vitamingruppen A, B, E, H und K und den Estern der vorgenannten Substanzen.
Zur Vitamin B-Gruppe oder zu dem Vitamin B-Komplex gehören insbesondere
- Vitamin B₁, Trivialname Thiamin, chemische Bezeichnung 3-[(4'-Amino-2'-methyl-5'-pyrimidinyl)-methyl]-5-(2-hydroxyethyl)-4-methylthiazoliumchlorid. Bevorzugt wird Thiaminhydrochlorid in Mengen von 0,05 bis 1 Gew.-%, bezogen auf die gesamte Zusammensetzung, eingesetzt.
- Vitamin B₂, Trivialname Riboflavin, chemische Bezeichnung 7,8-Dimethyl-10-(1-D-ribityl)-benzo[g]-pteridin-2,4(3*H*,10*H*)-dion. Bevorzugt werden Riboflavin oder seine Derivate in Mengen von 0,05 bis 1 Gew.-%, bezogen auf die gesamte Zusammensetzung, eingesetzt.
- Vitamin B₃. Unter dieser Bezeichnung werden die Verbindungen Nicotinsäure und Nicotinsäureamid (Niacinamid) geführt. Erfindungsgemäß bevorzugt ist das Nicotinsäureamid, das in den erfindungsgemäß verwendeten Zusammensetzungen bevorzugt in Mengen von 0,05 bis 1 Gew.-%, bezogen auf die gesamte Zusammensetzung, enthalten ist.
- Vitamin B₅ (Pantothensäure und Panthenol). Bevorzugt wird Panthenol eingesetzt. Erfindungsgemäß einsetzbare Derivate des Panthenols sind insbesondere die Ester und Ether des Panthenols sowie kationisch derivatisierte Panthenole. In einer weiteren bevorzugten Ausführungsform der Erfindung können an Stelle von sowie zusätzlich zu Pantothensäure oder Panthenol auch Derivate des 2-Furanon mit der allgemeinen Strukturformel (VIT-I) eingesetzt werden. 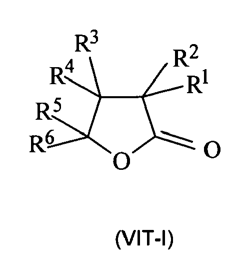 Bevorzugt sind die 2-Furanon-Derivate, in denen die Substituenten R¹ bis R⁶ unabhängig voneinander ein Wasserstoffatom, einen Hydroxylrest, einen Methyl-, Methoxy-, Aminomethyl- oder Hydroxymethylrest, einen gesättigten oder ein- oder zweifach ungesättigten, linearen oder verzweigten C₂-C₄ - Kohlenwasserstoffrest, einen gesättigten oder ein- oder zweifach ungesättigten, verzweigten oder linearen Mono-, Di- oder Trihydroxy-C₂-C₄ - Kohlenwasserstoffrest oder einen gesättigten oder ein- oder zweifach ungesättigten, verzweigten oder linearen Mono-, Di- oder Triamino-C₂-C₄ - Kohlenwasserstoffrest darstellen. Besonders bevorzugte Derivate sind die auch im Handel erhältlichen Substanzen Dihydro-3-hydroxy-4,4-dimethyl-2(3H)-furanon mit dem Trivialnamen Pantolacton (Merck), 4-Hydroxymethyl-γ-butyrolacton (Merck), 3,3-Dimethyl-2-hydroxy-γ-butyrolacton (Aldrich) und 2,5-Dihydro-5-methoxy-2-furanon (Merck), wobei ausdrücklich alle Stereoisomeren eingeschlossen sind. Das erfindungsgemäß außerordentlich bevorzugte 2-Furanon-Derivat ist Pantolacton (Dihydro-3-hydroxy-4,4-dimethyl-2(3H)-furanon), wobei in Formel (VIT-I) R¹ für eine Hydroxylgruppe, R² für ein Wasserstoffatom, R³ und R⁴ für eine Methylgruppe und R⁵ und R⁶ für ein Wasserstoffatom stehen. Das Stereoisomer (R)-Pantolacton entsteht beim Abbau von Pantothensäure.
   Die genannten Verbindungen des Vitamin B₅-Typs sowie die 2-Furanonderivate sind in den erfindungsgemäßen Mitteln in einer Gesamtmenge von 0,05 bis 5 Gew.-%, bevorzugt 0,1 bis 3 Gew.-%, besonders bevorzugt 0,5 bis 2 Gew.-%, jeweils bezogen auf die gesamte Zusammensetzung, enthalten.
- Vitamin B₆, wobei man hierunter keine einheitliche Substanz, sondern die unter den Trivialnamen Pyridoxin, Pyridoxamin und Pyridoxal bekannten Derivate des 5-Hydroxymethyl-2-methylpyridin-3-ols versteht. Vitamin B₆ ist in den erfindungsgemäß verwendeten Mitteln bevorzugt in Mengen von 0,0001 bis 1,0 Gew.-%, insbesondere in Mengen von 0,001 bis 0,01 Gew.-%, enthalten.
- Vitamin B₇ (Biotin), auch als Vitamin H oder "Hautvitamin" bezeichnet. Bei Biotin handelt es sich um (3a*S*,4*S*, 6a*R*)-2-Oxohexahydrothienol[3,4-*d*]-imidazol-4-valeriansäure. Biotin ist in den erfindungsgemäßen Mitteln bevorzugt in Mengen von 0,0001 bis 1,0 Gew.-%, insbesondere in Mengen von 0,001 bis 0,01 Gew.-% enthalten.
- Folsäure (Vitamin B₉, Vitamin B_{c}). Internationaler Freiname für N-[4-(2-Amino-3,4-dihydro-4-oxo-6-pteridinylmethylamino)-benzoyl]-L-glutaminsäure (N-Pteroyl-L-glutaminsäure, PteGlu). Folat wird synonym zu Pteroylglutamat gebraucht, Folate ist der Sammelbegriff für alle Folsäure-wirksamen Verbindungen und bezeichnet eine Substanzklasse, die einen mit 4-Aminobenzoesäure und L-Glutaminsäure verbundenen Pteridin-Ring enthält. Folsäure ist ein Wachstumsfaktor für verschiedene Mikroorganismen und eine Verbindung mit Vitamincharakter, die in der Natur meist als Polyglutamat und in reduzierter Form (7,8-Dihydrofolsäure, H₂Folat, DHF; Tetrahydrofolsäure, H₄Folat, THF; 5'-Methyl-Tetrahydrofolsäure, CH₃-H₄Folat, MeTHF) vorkommt. Erfindungsgemäß besonders bevorzugt verwendete Zusammensetzungen sind dadurch gekennzeichnet, dass sie mindestens eine Komponente, ausgewählt aus Folsäure, Folaten und deren Estern, in einer Gesamtmenge von 0,0001 bis 1,0 Gew.-%, insbesondere 0,01 bis 0,5 Gew.-%, bezogen auf die Zusammensetzung, enthalten.
- Orotsäure (Vitamin B₁₃, 1,2,3,6-Tetrahydro-2,6-dioxo-4-pyrimidin-carbonsäure, Uracil-6-carbonsäure, Molkensäure). Orotsäure, ihr Cholinester oder Orotsäure-Metallsalze (Orotate von Ca, Cr, Fe, K, Co, Cu, Li, Mg, Mn, Na, Zn, Sn) sind erfindungsgemäß besonders bevorzugt. Erfindungsgemäß besonders bevorzugt verwendete Zusammensetzungen sind dadurch gekennzeichnet, dass sie mindestens eine Komponente, ausgewählt aus Orotsäure, Orotaten und deren Estern, in einer Gesamtmenge von 0,0001 - 1,0 Gew.-%, insbesondere 0,01 - 0,5 Gew.-%, bezogen auf die Zusammensetzung, enthalten.

Zur Vitamin E-Gruppe zählen Tocopherol, insbesondere α-Tocopherol, und seine Derivate. Bevorzugte Derivate sind insbesondere die Ester, wie Tocopherylacetat, -nicotinat, -phosphat, -succinat, -linoleat, -oleat, Tocophereth-5, Tocophereth-10, Tocophereth-12, Tocophereth-18, Tocophereth-50 und Tocophersolan. Tocopherol und seine Derivate sind bevorzugt in Mengen von 0,05 - 1 Gew.-%, bezogen auf die gesamte Zusammensetzung, enthalten.
Vitamin H ist eine andere Bezeichnung für Biotin oder Vitamin B₇ (siehe oben).
Zu den fettlöslichen Vitaminen der Vitamin K-Gruppe, denen das Grundgerüst des 2-Methyl-1,4-naphthochinons zugrunde liegt, gehören Phyllochinon (Vitamin K₁), Farnochinon oder Menachinon-7 (Vitamin K2) und Menadion (Vitamin K₃). Vitamin K ist bevorzugt in Mengen von 0,0001 bis 1,0 Gew.-%, insbesondere 0,01 bis 0,5 Gew.-%, jeweils bezogen auf die gesamte Zusammensetzung, enthalten. Erfindungsgemäß besonders bevorzugt verwendete kosmetische oder dermatologische Zusammensetzungen zur topischen Behandlung der Haut sind dadurch gekennzeichnet, dass sie weiterhin mindestens einen kosmetischen Wirkstoff enthalten, der ausgewählt ist aus mindestens einer α-Hydroxycarbonsäure, α-Ketocarbonsäure oder β-Hydroxycarbonsäure oder deren Ester-, Lacton- und/oder Salzform. Erfindungsgemäß geeignete α-Hydroxycarbonsäuren oder α-Ketocarbonsäuren sind Glycolsäure, Milchsäure, Weinsäure, Citronensäure, 2-Hydroxybutansäure, 2,3-Dihydroxypropansäure, 2-Hydroxypentansäure, 2-Hydroxyhexansäure, 2-Hydroxyheptansäure, 2-Hydroxyoctansäure, 2-Hydroxydecansäure, 2-Hydroxydodecansäure, 2-Hydroxytetradecansäure, 2-Hydroxyhexadecansäure, 2-Hydroxyoctadecansäure, Mandelsäure, 4-Hydroxymandelsäure, Äpfelsäure, Erythrarsäure, Threarsäure, Glucarsäure, Galactarsäure, Mannarsäure, Gularsäure, 2-Hydroxy-2-methylbernsteinsäure, Gluconsäure, Brenztraubensäure, Glucuronsäure und Galacturonsäure. Besonders bevorzugte α-Hydroxycarbonsäuren sind Milchsäure, Citronensäure, Glycolsäure und Gluconsäure. Eine besonders bevorzugte β-Hydroxycarbonsäure ist Salicylsäure. Eine besonders bevorzugte α-Ketocarbonsäure ist Brenztraubensäure. Die Ester der genannten Säuren sind ausgewählt aus den Methyl-, Ethyl-, Propyl-, Isopropyl-, Butyl-, Amyl-, Pentyl-, Hexyl-, 2-Ethylhexyl-, Octyl-, Decyl-, Dodecyl- und Hexadecylestern. Weitere Derivate der vorgenannten Säuren sind deren physiologisch verträglichen Salze, bevorzugt die Zink-, Kupfer- und Mangansalze, die Salze der Alkali- und der Erdalkalimetalle und die Ammonium-, Alkylammonium-, Alkanolammonium- und Glucammoniumsalze, besonders bevorzugt die Natrium-, Kalium-, Magnesium- und Calcium-Salze.
Die α-Hydroxycarbonsäuren, α-Ketocarbonsäuren oder β-Hydroxycarbonsäuren oder ihre Derivate sind bevorzugt in einer Gesamtmenge von 0,1 - 10 Gew.-%, besonders bevorzugt 0,5 - 5 Gew.-% und außerordentlich bevorzugt 1 - 2 Gew.-%, jeweils bezogen auf die gesamte Zusammensetzung, enthalten.

Erfindungsgemäß besonders bevorzugt verwendete kosmetische oder dermatologische Zusammensetzungen zur topischen Behandlung der Haut sind dadurch gekennzeichnet, dass sie weiterhin mindestens einen kosmetischen Wirkstoff enthalten, der ausgewählt ist aus mindestens einem Flavonoid und/oder mindestens einem Flavonoid-reichen Pflanzenextrakt.
Die erfindungsgemäß bevorzugten Flavonoide umfassen die Glycoside der Flavone, der 3-Hydroxyflavone (Flavonole) und der Aurone. Die Flavanone und die Isoflavone sind erfindungsgemäß ausdrücklich von den Flavonoiden ausgenommen. Besonders bevorzugte Flavonoide sind ausgewählt aus Naringin (Aurantiin, Naringenin-7-rhamnoglucosid), α-Glucosylrutin, α-Glucosylmyricetin, α-Glucosylisoquercetin, α-Glucosylquercetin, Rutin (3,3',4',5,7-Pentahydroxyflavon-3-rhamnoglucosid, Quercetin-3-rhamnoglucosid), Troxerutin (3,5-Dihydroxy-3',4',7-tris(2-hydroxyethoxy)-flavon-3-(6-O-(6-deoxy-α-L-mannopyranosyl)-β-D-glucopyranosid)), Monoxerutin (3,3',4',5-Tetrahydroxy-7-(2-hydroxyethoxy)-flavon-3-(6-O-(6-deoxy-α-L-mannopyranosyl)-β-D-glucopyranosid)), Eriodictin und Apigenin-7-glucosid (4',5,7-Trihydroxyflavon-7-glucosid).
Erfindungsgemäß außerordentlich bevorzugte Flavonoide sind α-Glucosylrutin, Naringin und Apigenin-7-glucosid.

Ebenfalls bevorzugt sind die aus zwei Flavonoideinheiten aufgebauten Biflavonoide, die z. B. in Gingko-Arten vorkommen. Weitere bevorzugte Flavonoide sind die Chalkone, vor allem Phloricin und Neohesperidindihydrochalkon.

Erfindungsgemäß werden die Flavonoide in Mengen von 0,0001 bis 1 Gew.-%, bevorzugt 0,0005 bis 0,5 Gew.-% und besonders bevorzugt 0,001 bis 0,1 Gew.-%, jeweils bezogen auf die Flavonoidaktivsubstanz in der gesamten kosmetischen Zusammensetzung, eingesetzt.

Erfindungsgemäß besonders bevorzugt verwendete kosmetische oder dermatologische Zusammensetzungen zur topischen Behandlung der Haut sind dadurch gekennzeichnet, dass sie weiterhin mindestens einen kosmetischen Wirkstoff enthalten, der ausgewählt ist aus mindestens einem Ubichinon oder einem Ubichinol oder deren Derivaten. Ubichinole sind die reduzierte Form der Ubichinone. Die erfindungsgemäß bevorzugten Ubichinone weisen die Formel (UBI-I) auf: 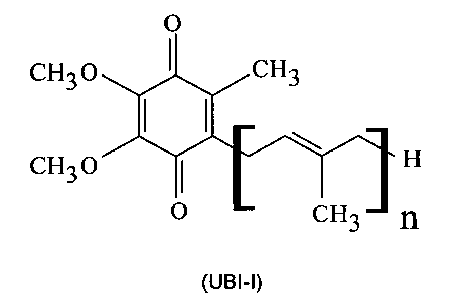 mit n = 6, 7, 8, 9 oder 10.
Besonders bevorzugt ist das Ubichinon der Formel (UBI-I) mit n = 10, auch bekannt als Coenzym Q10. Erfindungsgemäß werden die Ubichinone, Ubichinole oder deren Derivate in Mengen von 0,0001 bis 1 Gew.-%, bevorzugt 0,001 bis 0,5 Gew.-% und besonders bevorzugt 0,005 bis 0,1 Gew.-%, jeweils bezogen auf die gesamte Zusammensetzung, eingesetzt.

Erfindungsgemäß besonders bevorzugt verwendete kosmetische oder dermatologische Zusammensetzungen zur topischen Behandlung der Haut sind dadurch gekennzeichnet, dass sie weiterhin Silymarin enthalten. Silymarin stellt erfindungsgemäß ein früher als einheitliche Substanz angesehenes Wirkstoff-Konzentrat aus den Früchten der Mariendistel (Silybum marianum) dar. Die Hauptbestandteile des Silymarins sind Silybin (Silymarin I), Silychristin (Silymarin II) und Silydianin, die zur Gruppe der Flavanolignane gehören.

Erfindungsgemäß wird Silymarin in Mengen von 0,0001 bis 1 Gew.-%, bevorzugt 0,001 bis 0,5 Gew.-% und besonders bevorzugt 0,005 bis 0,1 Gew.-%, jeweils bezogen auf die gesamte Zusammensetzung, eingesetzt.

Erfindungsgemäß besonders bevorzugt verwendete kosmetische oder dermatologische Zusammensetzungen zur topischen Behandlung der Haut sind dadurch gekennzeichnet, dass sie weiterhin Ectoin enthalten. Ectoin ist der Trivialname für 2-Methyl-1,4,5,6-tetrahydropyrimidin-4-carboxylat. Erfindungsgemäß ist Ectoin bevorzugt in Mengen von 0,0001 bis 1 Gew.-%, besonders bevorzugt 0,001 bis 0,5 Gew.-% und außerordentlich bevorzugt 0,005 bis 0,01 Gew.-%, jeweils bezogen auf die gesamte Zusammensetzung, enthalten.
Erfindungsgemäß besonders bevorzugt verwendete kosmetische oder dermatologische Zusammensetzungen zur topischen Behandlung der Haut sind dadurch gekennzeichnet, dass sie weiterhin mindestens eine anorganische und/oder mindestens eine organische UV-Filtersubstanz enthalten.
Bei den UV-Filtersubstanzen handelt es sich um bei Raumtemperatur flüssig oder kristallin vorliegende Substanzen, die in der Lage sind, ultraviolette Strahlen zu absorbieren und die aufgenommene Energie in Form längerwelliger Strahlung, z. B. Wärme wieder abzugeben. Man unterscheidet UVA-Filter und UVB-Filter. Die UVA- und UVB-Filter können sowohl einzeln als auch in Mischungen eingesetzt werden. Der Einsatz von Filtermischungen ist erfindungsgemäß bevorzugt.
Die erfindungsgemäß bevorzugten organischen UV-Filter sind ausgewählt aus den Derivaten von Dibenzoylmethan, Zimtsäureestern, Diphenylacrylsäureestern, Benzophenon, Campher, p-Aminobenzoesäureestern, o-Aminobenzoesäureestern, Salicylsäureestern, Benzimidazolen, symmetrisch oder unsymmetrisch substituierten 1,3,5-Triazinen, monomeren und oligomeren 4,4-Diarylbutadiencarbonsäureestern und -carbonsäureamiden, Ketotricyclo(5.2.1.0)decan, Benzalmalonsäureestern, Benzoxazol sowie beliebigen Mischungen der genannten Komponenten. Die organischen UV-Filter können öllöslich oder wasserlöslich sein. Die Benzoxazol-Derivate liegen vorteilhaft in gelöster Form in den erfindungsgemäßen kosmetischen Zubereitungen vor. Es kann ggf. aber auch von Vorteil sein, wenn die Benzoxazol-Derivate in pigmentärer, d. h. ungelöster Form - beispielsweise in Partikelgrößen von 10 nm bis zu 300 nm - vorliegen. Erfindungsgemäß besonders bevorzugte öllösliche UV-Filter sind 1-(4-tert.-Butylphenyl)-3-(4'-methoxyphenyl)propan-1,3-dion (Parsol^{®} 1789), 1-Phenyl-3-(4'-isopropylphenyl)-propan-1,3-dion, 3-(4'-Methylbenzyliden)-D,L-campher, 4-(Dimethylamino)-benzoesäure-2-ethylhexylester, 4-(Dimethylamino)benzoesäure-2-octylester, 4-(Dimethylamino)-benzoesäure-amylester, 4-Methoxyzimtsäure-2-ethylhexylester, 4-Methoxyzimtsäurepropylester, 4-Methoxyzimtsäureisopentylester, 2-Cyano-3,3-phenylzimtsäure-2-ethylhexylester (Octocrylene), Salicylsäure-2-ethylhexylester, Salicylsäure-4-isopropylbenzylester, Salicylsäurehomomenthylester (3,3,5-Trimethyl-cyclohexylsalicylat), 2-Hydroxy-4-methoxybenzophenon, 2-Hydroxy-4-methoxy-4'-methylbenzophenon, 2,2'-Dihydroxy-4-methoxybenzophenon, 2-(4'-Diethylamino-2'-hydroxybenzoyl)-benzoesäurehexylester (auch: Aminobenzophenon, unter der Bezeichnung Uvinul A Plus bei der Firma BASF erhältlich), 4-Methoxybenzmalonsäuredi-2-ethylhexylester, an Polymere gebundene UV-Filter, z. B. das 3-(4-(2,2-Bis-Ethoxycarbonylvinyl)-phenoxy)propenyl)-methoxysiloxan/Dimethylsiloxan-Copolymer mit der INCI-Bezeichnung Dimethicodiethylbenzal malonate (CAS-Nr. 207574-74-1, Parsol^{®} SLX), Triazinderivate, wie z. B. 2,4-Bis-{[4-(2-Ethyl-hexyloxy)-2-hydroxy]-phenyl}-6-(4-methoxyphenyl)-1,3,5-triazin (INCI: Bis-Ethylhexyloxyphenol Methoxyphenyl Triazin, unter dem Namen Tinosorb S bei CIBA erhältlich), Dioctylbutylamidotriazon (INCI: Diethylhexyl Butamido Triazone, unter dem Namen Uvasorb^{®} HEB bei Sigma 3V erhältlich), 2,4,6-Trianilino-(p-carbo-2'-ethyl-1'-hexyloxy)-1,3,5-triazin (Ethylhexyl Triazone, Uvinul^{®} T 150), 2-[4,6-Bis(2,4-dimethylphenyl)-1,3,5-triazin-2-yl]-5-(octyloxy)phenol (CAS Nr.: 2725-22-6), 2,4-bis-[5-1(di-methylpropyl)benzoxazol-2-yl-(4-phenyl)-imino]-6-(2-ethylhexyl)-imino-1,3,5-triazin (CAS Nr. 288254-16-0, Uvasorb^{®} K2A von 3V Sigma), die Benzotriazolderivate 2,2'-Methylen-bis-(6-(2H-benzotriazol-2-yl)-4-(1,1,3,3-tetramethylbutyl)-phenol) [Tinosorb M (Ciba)], 2,2'-Methyl-bis-[6(2H-benzotriazol-2-yl)-4-(methyl)phenol] (MIXXIM BB/200 der Firma Fairmount Chemical), 2-(2'-Hydroxy-3',5'-di-t-amylphenyl)benzotriazol (CAS- Nr.: 025973-551), 2-(2'-Hydroxy-5'-octylphenyl)-benzotriazol (CAS-Nr. 003147-75-9), 2-(2'-Hydroxy-5'-methylphenyl)benzotriazol (CAS-Nr. 2440-22-4), 2-(2H-benzotriazol-2-yl)-4-methyl-6-[2-methyl-3-[1,3,3,3-tetramethyl-1-((trimethylsilyl)oxy]disiloxanyl)propyl]-phenol (CAS-Nr.: 155633-54-8) mit der INCI-Bezeichnung Drometrizole Trisiloxane, 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin oder auch Aniso Triazin, erhältlich als Tinosorb^{®} S von CIBA), 2,4-Bis-{[4-(3-sulfonato)-2-hydroxy-propyloxy)-2-hydroxy]-phenyl}-6-(4-methoxyphenyl)-1,3,5-triazin-Natriumsalz, 2,4-Bis-{[4-(3-(2-propyloxy)-2-hydroxy-propyloxy)-2-hydroxy]-phenyl}-6-(4-methoxyphenyl)-1,3,5-triazin, 2,4-Bis-{[4-(2-ethyl-hexyloxy)-2-hydroxy]-phenyl}-6-[4-(2-methoxyethylcarboxyl)-phenylamino]-1,3,5-triazin, 2,4-Bis-{[4-(3-(2-propyloxy)-2-hydroxy-propyloxy)-2-hydroxy]-phenyl}-6-[4-(ethylcarboxyl)-phenylamino]-1,3,5-triazin, 2,4-Bis-{[4-(2-ethylhexyloxy)-2-hydroxy]-phenyl}-6-(1-methyl-pyrrol-2-yl)-1,3,5-triazin, 2,4-Bis-{[4-tris(trimethylsiloxy-silylpropyloxy)-2-hydroxy]-phenyl}-6-(4-methoxyphenyl)-1,3,5-triazin, 2,4-Bis-{[4-(2-methylpropenyloxy)-2-hydroxy]-phenyl}-6-(4-methoxyphenyl)-1,3,5-triazin, 2,4-Bis-{[4-(1',1',1',3',5',5',5'-Heptamethylsiloxy-2-methyl-propyloxy)-2-hydroxy]-phenyl}-6-(4-methoxyphenyl)-1,3,5-triazin sowie Mischungen der genannten Komponenten.

Bevorzugte wasserlösliche UV-Filter sind 2-Phenylbenzimidazol-5-sulfonsäure, Phenylen-1,4-bis-(2-benzimidazyl)-3,3'-5,5'-tetrasulfonsäure und deren Alkali-, Erdalkali-, Ammonium-, Alkylammonium-, Alkanolammonium- und Glucammoniumsalze, insbesondere die Sulfonsäure selbst mit der INCI-Bezeichnung Phenylbenzimidazole Sulfonic Acid (CAS.-Nr. 27503-81-7), die beispielsweise unter dem Handelsnamen Eusolex 232 bei Merck oder unter Neo Heliopan Hydro bei Symrise erhältlich ist, und das Phenylen-1,4-bis-(2-benzimidazyl)-3,3'-5,5'-tetrasulfonsäure-bis-natriumsalz mit der INCI-Bezeichnung Disodium Phenyl Dibenzimidazol Tetrasulfonate (CAS-Nr.: 180898-37-7), das beispielsweise unter dem Handelsnamen Neo Heliopan AP bei Symrise erhältlich ist, Sulfonsäurederivate von Benzophenonen, vorzugsweise 2-Hydroxy-4-methoxybenzophenon-5-sulfonsäure und ihre Salze, Sulfonsäurederivate des 3-Benzylidencamphers, wie z. B. 4-(2-Oxo-3-bornylidenmethyl)benzolsulfonsäure und 2-Methyl-5-(2-oxo-3-bornyliden)sulfonsäure und deren Salze mit der INCI-Bezeichnung Terephthalydene Dicampher Sulfonic Acid (CAS.-Nr.: 90457-82-2, als Mexoryl SX von der Firma Chimex erhältlich).
Einige der öllöslichen UV-Filter können selbst als Lösungsmittel oder Lösungsvermittler für andere UV-Filter dienen. So lassen sich beispielsweise Lösungen des UV-A-Filters 1-(4-tert.-Butylphenyl)-3-(4'methoxyphenyl)propan-1,3-dion (z. B. Parsol^{®} 1789) in verschiedenen UV-B-Filtern herstellen. Die erfindungsgemäßen Zusammensetzungen enthalten daher in einer weiteren bevorzugten Ausführungsform 1-(4-tert.-Butylphenyl)-3-(4'-methoxyphenyl)propan-1,3-dion in Kombination mit mindestens einem UV-B-Filter, ausgewählt aus 4-Methoxyzimtsäure-2-ethylhexylester, 2-Cyano-3,3-phenylzimtsäure-2-ethylhexylester, Salicylsäure-2-ethylhexylester und 3,3,5-Trimethyl-cyclohexylsalicylat. In diesen Kombinationen liegt das Gewichtsverhältnis von UV-B-Filter zu 1-(4-tert.-Butylphenyl)-3-(4'-methoxyphenyl)propan-1,3-dion zwischen 1:1 und 10:1, bevorzugt zwischen 2:1 und 8:1, das molare Verhältnis liegt entsprechend zwischen 0,3 und 3,8, bevorzugt zwischen 0,7 und 3,0.
Bei den erfindungsgemäß bevorzugten anorganischen Lichtschutzpigmenten handelt es sich um feindisperse oder kolloiddisperse Metalloxide und Metallsalze, beispielsweise Titandioxid, Zinkoxid, Eisenoxid, Aluminiumoxid, Ceroxid, Zirkoniumoxid, Silicate (Talk) und Bariumsulfat. Die Partikel sollten dabei einen mittleren Durchmesser von weniger als 100 nm, vorzugsweise zwischen 5 und 50 nm und insbesondere zwischen 15 und 30 nm aufweisen, so genannte Nanopigmente. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z. B. Titandioxid T 805 (Degussa) oder Eusolex^{®} T2000 (Merck). Als hydrophobe Coatingmittel kommen dabei vor allem Silicone und dabei speziell Trialkoxyoctylsilane oder Simethicone in Frage. Besonders bevorzugt sind Titandioxid und Zinkoxid.
Erfindungsgemäß ist mindestens eine organische UV-Filtersubstanz bevorzugt in einer Gesamtmenge von 0,1 - 30 Gew.-%, besonders bevorzugt 0,5 - 20 Gew.-%, außerordentlich bevorzugt 1,0 - 15 Gew.-% und weiter bevorzugt 3,0 - 10 Gew.-%, jeweils bezogen auf die gesamte Zusammensetzung, enthalten.
Erfindungsgemäß ist mindestens eine anorganische UV-Filtersubstanz bevorzugt in einer Gesamtmenge von 0,1 - 15 Gew.-%, besonders bevorzugt 0,5 - 10 Gew.-%, außerordentlich bevorzugt 1,0 - 5 Gew.-% und weiter bevorzugt 2,0 - 4,0 Gew.-%, jeweils bezogen auf die gesamte Zusammensetzung, enthalten.

Erfindungsgemäß besonders bevorzugt verwendete kosmetische oder dermatologische Zusammensetzungen zur topischen Behandlung der Haut sind dadurch gekennzeichnet, dass sie weiterhin mindestens einen selbstbräunenden Wirkstoff enthalten. Erfindungsgemäß bevorzugte selbstbräunende Wirkstoffe sind ausgewählt aus Dihydroxyaceton, Erythrulose und 5,6-Dihydroxyindolin.
Erfindungsgemäß ist der mindestens eine selbstbräunende Wirkstoff in einer Gesamtmenge von 0,01 - 15 Gew.-%, bevorzugt 0,1 - 10 Gew.-%, besonders bevorzugt 1,0 - 5 Gew.-% und außerordentlich bevorzugt 2,0 - 4,0 Gew.-%, jeweils bezogen auf die gesamte Zusammensetzung, enthalten.

Erfindungsgemäß besonders bevorzugt verwendete kosmetische oder dermatologische Zusammensetzungen zur topischen Behandlung der Haut sind dadurch gekennzeichnet, dass sie weiterhin mindestens einen hautberuhigenden Wirkstoff enthalten. Erfindungsgemäß bevorzugte hautberuhigende Wirkstoffe sind ausgewählt aus Allantoin, α-Bisabolol, α-Liponsäure, Extrakten aus Centella asiatica, beispielsweise erhältlich unter der Bezeichnung Madecassicoside von DSM, Glycyrrethinsäure, die besonders bevorzugt in Liposomen verkapselt vorliegt und in dieser Form z. B. unter dem Handelsnamen Calmsphere von Soliance erhältlich ist, Mischungen aus Getreidewachsen, Extrakten aus Schibutter und Argania Spinosa-Öl mit der INCl-Bezeichnung "Spent grain wax and Butyrospermum Parkii (shea butter) extract and Argania Spinosa Kernel Oil", wie sie z. B. unter der Handelsbezeichnung Stimu-Tex AS von der Firma Pentapharm erhältlich sind, Extrakten aus Vanilla Tahitensis, wie sie z. B. unter der Handelsbezeichnung Vanirea (INCl : Vanilla Tahitensis Fruit Extract) von der Firma Solabia erhältlich sind, Alginhydrolysaten, wie sie z. B. unter der Handelsbezeichnung Phycosaccharide, insbesondere Phycosaccharide Al, von der Firma Codif erhältlich sind, Extrakten aus Bacopa Monniera, wie sie z. B. unter der Handelsbezeichnung Bacocalmine von der Firma Sederma erhältlich sind, Extrakten aus der Rooibos-Pflanze, wie sie z. B. unter dem Handelsnamen Rooibos Herbasec MPE von der Firma Cosmetochem erhältlich sind, Hefeextrakten, besonders bevorzugt das Handelsprodukt Drieline (INCI-Bezeichnung "Sorbitol, Yeast Extract"), erhältlich von der Firma Lanatech, den physiologisch verträglichen Salzen von Sterolsulfaten, wie sie z. B. unter der Handelsbezeichnung Phytocohesine (INCl: Sodium Beta-Sitosterylsulfate) von der Firma Vincience erhältlich sind, Aminodicarbonsäuren mit einer C-Kettenlänge von 3 - 6 Kohlenstoffatomen sowie deren physiologisch verträglichen Salzen, bevorzugt ausgewählt aus Aminomalonsäure, Aminobernsteinsäure (= Asparaginsäure), Aminoglutarsäure und Aminoadipinsäure sowie deren physiologisch verträglichen Salzen wie Kaliumaspartat und Magnesiumaspartat, sowie beliebigen Mischungen dieser Substanzen.
Der mindestens eine hautberuhigende Wirkstoff ist bevorzugt in einer Gesamtmenge von 0,001 bis 5 Gew.-%, besonders bevorzugt 0,01 bis 2 Gew.-% und außerordentlich bevorzugt 0,1 bis 1 Gew.-%, jeweils bezogen auf die gesamte Zusammensetzung, enthalten.

Erfindungsgemäß besonders bevorzugt verwendete kosmetische oder dermatologische Zusammensetzungen zur topischen Behandlung der Haut, sind dadurch gekennzeichnet, dass sie weiterhin mindestens einen feuchtigkeitsspendenden Wirkstoff enthalten. Erfindungsgemäß bevorzugte feuchtigkeitsspendende Wirkstoffe sind ausgewählt aus Desoxyzuckern, besonders bevorzugt Rhamnose und Fucose, Desoxyzucker-Bausteine enthaltende Polysaccharide, besonders bevorzugt Biosaccharide Gum-1, enthalten beispielsweise in dem Handelsprodukt Fucogel^{®} von Solabia Biosaccharide Gum-2, enthalten beispielsweise in dem Handelsprodukt Rhamnosoft^{®} von Solabia, Biosaccharide Gum-3, enthalten beispielsweise in dem Handelsprodukt Fucogenol^{®} von Solabia, Biosaccharide Gum-4, enthalten beispielsweise in dem Handelsprodukt Glycofilm^{®} von Solabia, Mischungen aus Biosaccharide Gum-2 und Biosaccharide Gum-3, beispielsweise erhältlich als Handelsprodukt Elastinol plus^{®} von Solabia, weiterhin Harnstoff, (2-Hydroxyethyl)harnstoff, N,N'-Bis-(2-hydroxyethyl)harnstoff, Betain (Me₃N⁺-CH₂-COO⁻), Glycosaminoglycanen, besonders bevorzugt Hyaluronsäure, Dextran, Dextransulfat, Chondroitin-4-sulfat und Chondroitin-6-sulfat.

Der mindestens eine feuchtigkeitsspendende Wirkstoff ist bevorzugt in einer Gesamtmenge von 0,001 bis 10 Gew.-%, besonders bevorzugt 0,01 bis 5 Gew.-% und außerordentlich bevorzugt 0,1 bis 1 oder 2 Gew.-%, jeweils bezogen auf die gesamte Zusammensetzung, enthalten.

Erfindungsgemäß besonders bevorzugt verwendete kosmetische oder dermatologische Zusammensetzungen zur topischen Behandlung der Haut sind dadurch gekennzeichnet, dass sie weiterhin mindestens einen sebumregulierenden Wirkstoff. Erfindungsgemäß bevorzugte sebumregulierende Wirkstoffe sind ausgewählt aus Azelainsäure, Sebacinsäure, 10-Hydroxydecansäure, 1,10-Decandiol, die als erfindungsgemäß besonders bevorzugte Dreierkombination in dem Handelsprodukt Acnacidol PG von Vincience eingesetzt werden, weiterhin aus dem Handelsprodukt Azeloglicina (Potassium Azeloyl Diglycinate) von Sinerga, Extrakten aus Spiraea Ulmaria, wie sie z. B. im Produkt Seboregul der Firma Silab enthalten sind, weiterhin aus wasser- und öllöslichen Extrakten aus Hamamelis, Klettenwurzel und Brennessel, Zimtbaumextract (z. B. Sepicontrol^{®} A5 von der Firma Seppic), Chrysanthemenextrakt (z. B. Laricyl^{®} von Laboratoires Sérobiologiques) und den Wirkstoffmischungen Asebiol^{®} BT 2 (Laboratoires Sérobiologiques, INCl: Aqua, Hydrolyzed Yeast Protein, Pyridoxine, Niacinamide, Glycerin, Panthenol, Allantoin, Biotin) und Antifettfaktor^{®} COS-218/2-A (Cosmetochem, INCl: Aqua, Cetyl-PCA, PEG-8 Isolauryl Thioether, PCA, Cetyl Alcohol).
Der mindestens eine sebumregulierende Wirkstoff ist bevorzugt in einer Gesamtmenge von 0,001 - 5 Gew.-%, besonders bevorzugt 0,01 - 2 Gew.-% und außerordentlich bevorzugt 0,1 - 1 Gew.-%, jeweils bezogen auf die gesamte Zusammensetzung, enthalten.

### Experimenteller Teil

### 1. CLBT-Verfahren zur Bestimmung der "complexion radiance"

Das CLBT-Verfahren basiert auf der visuellen Begutachtung des Erscheinungsbildes der Haut durch ein trainiertes Gutachtergremium. Die folgenden Parameter werden bestimmt: Farbe, Brillanz, Glanz und Transparenz (C.L.B.T.: colour, luminosity, brightness, transparency). Bei dem Parameter Farbe werden folgende Farben näher beurteilt: "rot-pink", "beige", "olive" und "hell-pink". Hierbei soll der Wert für "rot-pink", "beige", "olive" sinken und für "hell-pink" steigen. Die Parameter Brillanz, Glanz und Transparenz sollen zunehmen.
Für alle vier Parameter stehen den Gutachtern Vergleichsskalen zur Verfügung (Skin Research and Technology, 2004, 10: 50-56).
Die Studie erfolgte an 20 Probanden im Alter von 24-35 Jahren über einen Zeitraum von 28 Tagen. Bei der Studie handelte es sich um eine offene Studie, so dass die Probanden als ihre eigene Referenz fungierten. Die Produkte, die eine Kombination aus Kombucha und Glistin enthielten, wurden zweimal täglich aufgetragen. Die oben genannten Parameter wurden von drei Gutachtern unter standardisierten Bedingungen zum Zeitpunkt T0 und T28 bewertet.

### Ergebnisse:

| | Farben (% Sättigung) | | | | L.B.T. (Grad/10) | | |
|---|---|---|---|---|---|---|---|
| | Pink-rot | Olive | Beige | Hell-pink | Brillanz | Glanz | Transparenz |
| T28 vs T0 | -1,75* | -2,54* | -2,28* | +1,75* | +1,75* | +0,18* | +0,02 |

| | | | | | | | |
|---|---|---|---|---|---|---|---|
| * signifikant nach Student t Test oder Wilcoxon Test | | | | | | | |

### 2. Allgemeine Bedingungen für in vitro Versuche

### 2.1 Zellkultur und UV-Belastung

Dermale Fibroblasten (isoliert aus menschlicher Vorhaut) wurden bei 37°C und 5 Vol.-% CO₂ mit Dulbecco's Minimal Essential Medium High Glucose unter Zusatz von 1 Gew.-% Glutamax, 1 Gew.-% Penicillin/Streptomycin und 10 Gew.-% fetalem Kälberserum kultiviert. Zur UV-A Bestrahlung mittels eines Teilkörperbestrahlungshimmels Typ Sellamed 2000 (Dr. Sellmeier; Bestrahlungsspektrum: 340-400 nm, Maximum: 360-380 nm) wurden die Zellen in Petrischalen ausplattiert (ca. 3,8 x 10⁴ Zellen/cm²; zuzüglich 2 Tage Wachstum, danach noch subkonfluent), das Zellmedium abgesaugt, die Zellen einmal mit PBS gewaschen und die UV-A Bestrahlung anschließend in PBS durchgeführt (Dosierung über Zeitintervall bis zu 14min bei 120J/cm²). Nach UV-A Bestrahlung und Entfernung des PBS wurden die Zellen wieder mit Zellmedium versetzt und bis zur Zellernte bei 37°C, 5 Vol.-% CO₂ aufbewahrt.
Nach Zelltrypsinierung und -schabung erfolgte unter Zusatz von hypotonem Salzpuffer (10 mM HEPES, 24 mM KCl, 10 mM MgCl₂, pH 7,5) und Anwendung der Frier-Tau-Technik die Herstellung des Zelllysats. Nicht lysierte Zellen sowie Membranen und Zellkerne wurden durch zehnminütige Zentrifugation bei 14000 rpm und 4°C abgetrennt.

Getestete Substanzen:
- Natriumascorbylphosphat (0,025 mg/ml), BASF, entsprechend einem Aktivsubstanzgehalt in der Testlösung von 0,0025 Gew.-%
- Hypotaurin 0,25 (mg/ml), Siber Hegner (entsprechend einem Aktivsubstanzgehalt in der Testlösung von 0,025 Gew.-%)
- Kombuchka (7,5 mg/ml), Sederma (entsprechend einem Aktivsubstanzgehalt in der Testlösung von 0,02 - 0,053 Gew.-%)
- Glistin (0,5 mg/ml), Exsymol (entsprechend 4,5 - 5,5' 10⁻⁴ Gew.-% Glutamylamidoethylindol in der Testlösung)
- Gingkoselect Phytosome (0,15 mg/ml), Indena (entsprechend 0,9 - 1,8 · 10⁻⁵ Gew.-% Ginsenoside-Aktivsubstanz in der Testlösung), wird vom Hersteller als Antioxidans ausgelobt.

### 3. Proteincarbonyle in vitro

Fibroblasten wurden für 16 Stunden mit Antioxidantien-haltigem Medium versetzt. Nach UV-A Bestrahlung in PBS wurden die Fibroblasten anschließend wieder für 3 Stunden mit Antioxidantien-haltigem Medium versetzt und lysiert. Die Detektion der Proteincarbonyle erfolgte per ELISA Technik nach Buss et al. mit Veränderungen nach Sitte et al. [6]. Das Zelllysat wurde mit Dinitrophenylhydrazin versetzt, und die gebildeten Hydrazone wurden mit Hilfe des primären Antikörpers Anti-Dinitrophenyl-Rabbit-IgG detektiert. Als sekundärer Antikörper wurde der Anti-Rabbit-IgG-Antikörper (Peroxidase-konjugiert) verwendet und die Menge des gebundenen, sekundären Antikörpers nach Entwicklung mit o-Phenylendiamin und Wasserstoffperoxid detektiert.

### Ergebnisse:

| | bestrahlt | | unbestrahlt | |
|---|---|---|---|---|
| | Proteincarbonyl-Gehalt [%, relativ zum unbehandelten und unbestrahlten Zustand] | Standardabweichung | Proteincarbonyl-Gehalt [%, relativ zum unbehandelten und unbestrahlten Zustand] | Standardabweichung |
| unbehandelt | 171,43 | 66,89 | 100 | 0 |
| Glistin | 139,96 | 55,17 | 112,69 | 17,94 |
| Kombuchka | 161,73 | 86,34 | 86,82 | 47,78 |
| Hypotaurin | 151,3 | 45,54 | 128,32 | 54,38 |
| Natriumascorbylphosphat | 124,18 | 40,67 | 86,82 | 52,52 |
| Gingkoselect Phytosome | 193,36 | 109,3 | 187,39 | |

Der Proteincarbonyl-Gehalt in den unbehandelten und unbestrahlten Fibroblasten wird mit 100 % angesetzt.

Bei Bestrahlung der unbehandelten Fibroblasten erhöht sich der Proteincarbonyl-Gehalt um mehr als 50%.

Die Ergebnisse zeigen, dass Glutamylamidoethylindol, Kombucha, Hypotaurin und Natriumascorbylphosphat den durch UV-A-Bestrahlung induzierten Anstieg des Proteincarbonyl-Gehalts reduzieren.

### 4. Proteasomaktivität

Die Behandlung der Fibroblasten mit Antioxidantien erfolgte wie unter 2.3.3. Das Zelllysat wurde mit einem Puffer, der Tris-HCl (pH=7,8 bei 37°C), KCI, Mg-Acetat, MgCl₂ und Dithiothreitol enthält, versetzt. Nach Zugabe von 10µl einer 2mM Stammlösung von suc-LLVY-MCA (N-Succinyl-Leucin-Leucin-Valin-Tyrosin-4-Methyl-cumaryl-7-amid) erfolgte eine 30-minütige Inkubation bei 37°C. Anschließend wurde die Fluoreszenz des Hydrolyseprodukts MCA (4-Methyl-cumaryl-7-amid) am Fluorimeter bestimmt (Anregungswellenlänge: 390 nm, Detektionswellenlänge: 460nm) und die MCA-Konzentration unter Verwendung einer Standardreihe bestimmt.

### Ergebnisse :

| | bestrahlt | | unbestrahlt | |
|---|---|---|---|---|
| | MW (%) | SD | MW (%) | SD |
| unbehandelt | 23,41 | 5,4 | 100 | 8,2 |
| Natriumascorbylphosphat | 33,36 | 2,92 | 104,74 | 12,43 |
| Hypotaurin | 32,9 | 1,98 | 144,37 | 1,95 |
| Kombuchka | 43,1 | 3,67 | 130,79 | 8,2 |
| Glistin | 24,94 | 1,83 | 119,32 | 5,32 |
| Gingkoselect Phytosome | 9,29 | 0,43 | 58,73 | 0,74 |

Der Proteasomaktivität in den unbehandelten und unbestrahlten Fibroblasten wird mit 100 % angesetzt.

Bei Bestrahlung der unbehandelten Fibroblasten sank die Proteasomaktivität auf 23,41 % gegenüber der Aktivität ohne Strahlungseinfluss.

Die Ergebnisse zeigen, dass Glutamylamidoethylindol, Kombucha, Hypotaurin und Natriumascorbylphosphat die durch UV-A-Bestrahlung induzierte Abnahme der Proteasomaktivität reduzieren.

### 5. Untersuchungen am Ganzhautmodell

Durch immunohistochemische Nachweismethoden konnte an Hautmodellen ein Anstieg der Lamininsynthese durch den Einfluss von Placebo + 3 Gew.-% Kombuchka gezeigt werden.

### 6. In vivo Untersuchungen

### Probandenauswahl

Es wurden 20 gesunde Probanden im Alter zwischen 35-65 Jahren untersucht. Je Proband wurden 5 Gewebeproben entnommen.

in vivo getestete Substanzen

### A Vehikel

| | |
|---|---|
| Montanov L | 3,50 |
| Capryl-/Caprinsäure-Triglycerid | 9,50 |
| Stenol 1618 | 1,50 |
| Cutina MD | 2,10 |
| Propylparaben | 0,20 |
| Hexandiol-1,6 | 7,00 |
| Glycerin 86% pflanzlich | 2,00 |
| Methylparaben | 0,20 |
| Tego Carbomer 140 | 0,20 |
| Natriumhydroxid Perlen | 0,02 |
| Wirkstoffe | entweder 1 Gew.-% Natriumascorbylphosphat oder 3 Gew.-% Kombuchka und 2 Gew.-% Glistin |
| Wasser, vollentsalzt | ad 100,00 |

B Vehikel mit 1 Gew.-% Natriumascorbylphosphat (=1. Aktivpräparat)
C Vehikel mit 3 Gew.-% Kombuchka und 2 Gew.-% Glistin (=2. Aktivpräparat)

### Probengewinnung

Nach Auftragen der Testsubstanzen im Gesäßbereich und einer Einwirkzeit von 20 Minuten wurden die entsprechenden Testareale mit 100 J/cm² (Bestrahlungsdauer ca. 40min) bestrahlt. 1 Stunde nach Ende der Bestrahlung erfolgte die Entnahme der Proben.

Behandlung der 5 Testareale:
- 2 Areale mit vorheriger/anschließender Applikation der 2 Aktivpräparate und UV-A Bestrahlung
- 1 Areal mit vorheriger/anschließender Applikation der Grundlagensubstanz (= Vehikel) und UV-A Bestrahlung
- 1 Areal mit ausschließlicher UV-A Bestrahlung (Positivkontrolle)
- 1 Areal, das nicht bestrahlt wurde (Negativkontrolle)

Die Hautbiopsien wurden nach Entnahme in zwei ungefähr gleich große Teile geschnitten und in flüssigen Stickstoff bis zur Probenauswertung aufbewahrt. Eine Hälfte wurde für biochemische Untersuchungen (Messung der Proteincarbonyle mittels ELISA, Messung der Proteasomaktivität) verwendet, die andere für immunhistochemische Untersuchungen zur Messung des Proteasomgehalts und der oxidierten Proteine verwendet.
Die Probenhomogenisation für biochemische Untersuchungen erfolgte durch Pottern der Biopsien in PBS. Anschließend wurden die Proben für 10min bei 14000 rpm und 4°C zentrifugiert und der Überstand für weitere Untersuchungen verwendet.

### 7. Proteincarbonyle in vitro

Bestimmungsmethode siehe oben, unter Abschnitt 3.

### Ergebnisse:

| | MW (%) | Standardabweichung |
|---|---|---|
| Placebo | 123 | 50 |
| Placebo + 1 Gew.-% Natriumascorbylphosphat | 113 | 34 |
| Placebo + 3 Gew.-% Kombuchka + 2 Gew.-% Glistin | 88 | 43 |
| unbehandelt, bestrahlt | 141 | 42 |
| unbehandelt, unbestrahlt | 100 | 28 |

Obwohl die Zusammensetzung mit Kombuchka und Glistin mit insgesamt ca. 0,23 Gew.-% Aktivsubstanz einen deutlich geringeren Aktivsubstanzgehalt aufweist als die Zusammensetzung mit Natriumascorbylphosphat, ist sie zur Reduzierung der schädlichen Proteincarbonyle deutlich besser geeignet.

### 8. Proteasomenaktivität in vivo

Bestimmungsmethode siehe oben, unter Abschnitt 4.

### Ergebnisse:

| | MW (%) | Standardabweichung |
|---|---|---|
| Placebo | 69,35 | 19,34 |
| Placebo + 1 Gew.-% Natriumascorbylphosphat | 83,27 | 40,47 |
| Placebo + 3 Gew.-% Kombuchka + 2 Gew.-% Glistin | 53,59 | 17,11 |
| unbehandelt, bestrahlt | 58,6 | 19,34 |
| unbehandelt, unbestrahlt | 100 | 0 |

### 9. Immunhistochemie

### 9.1 Gewinnung der Schnitte

Die Hautbiopsien wurden mit Hilfe eines Cryotoms 6µm breit geschnitten und bis zur immunhistochemischen Färbung bei -80°C gelagert.

### 9.2 Proteinoxidation

Die immunhistochemische Detektion der Proteincarbonyle erfolgte nach einem modifizierten Protokoll von Smith et al.. Die Fixierung der Gewebeschnitte zur Entfernung der Lipidreste wurde mit Methacarn (Methanol: Chloroform: Essigsäure/ 60:30:10) für 24h bei 4°C durchgeführt. Nach Entfernung des Methacarns wurden die Zellen für 16h bei 4°C mit DNPH (300mg DNPH in 100ml von 96 % Ethanol, mit Zusatz von 1,5% Schwefelsäure) versetzt. Die gebildeten Hydrazone wurden mittels anti-DNP-Antikörpers (Verdünnung 1:200) detektiert nach Waschen der Schnitte und Blocken für 30 min bei 4°C mit PBS (mit 1% fetalem Kälberserum). Nach Zugabe des primären Antikörpers für 1h bei 4°C wurden die Schnitte gewaschen und der TRITC gelabelte, sekundäre Antikörper (Verdünnung 1:100) für 1h bei 4°C zugegeben. Nach Entfernung des sekundären Antikörpers wurden die Schnitte gewaschen und fluoreszenzmikroskopisch untersucht.

### Ergebnisse:

| | MW (%) | Standardabweichung |
|---|---|---|
| Placebo | 146,59 | 11,56 |
| Placebo + 1 Gew.-% Natriumascorbylphosphat | 138,69 | 14,48 |
| Placebo + 3 Gew.-% Kombuchka + 2 Gew.-% Glistin | 112,13 | 11,84 |
| unbehandelt, bestrahlt | 151,77 | 12,69 |
| unbehandelt, unbestrahlt | 100 | 0 |

Die Studien an Hautbiopsien bestätigen die in-vitro-Ergebnisse, dass die Zusammensetzung mit Kombuchka und Glistin die schädlichen Proteincarbonyle wirksamer reduziert als die Zusammensetzung mit Natriumascorbylphosphat.

Die folgenden Formulierungsbeispiele sollen den Gegenstand der Erfindung näher erläutern, ohne ihn hierauf zu beschränken.

### 1. Öl-in-Wasser-Emulsionen

### 1. Beispielserie: Tagescremes mit Repair-Effekt auf Zellebene

| | 1 | 2 mit "complexion radiance"-Effekt | 3 mit "complexion radiance"-Effekt | 4 mit "complexion radiance"-Effekt |
|---|---|---|---|---|
| Distelöl | 3,00 | 3,00 | 3,00 | 3,00 |
| Myritol 318 | 5,00 | 5,00 | 5,00 | 5,00 |
| Novata AB | 2,00 | 2,00 | 2,00 | 2,00 |
| Lanette 22 | 1,00 | 1,00 | 1,00 | 1,00 |
| Cutina MD | 2,00 | 2,00 | 2,00 | 2,00 |
| Stenol 1618 | 1,00 | 1,00 | 1,00 | 1,00 |
| Baysilon M350 | 1,00 | 1,00 | 1,00 | 1,00 |
| Controx KS | 0,05 | 0,05 | 0,05 | 0,05 |
| Propylparaben | 0,20 | 0,20 | 0,20 | 0,20 |
| Dry Flo Plus | 1,00 | 2,00 | 3,00 | 2,00 |
| Propylenglycol | 5,00 | 5,00 | 5,00 | 5,00 |
| Glycerin | 5,00 | 3,00 | 3,00 | 3,00 |
| Methylparaben | 0,20 | 0,20 | 0,20 | 0,20 |
| Phenoxyethanol | 0,9 | 0,5 | 0,9 | 0,5 |
| Herbasol Destillat Salbei | 1,0 | - | 1,0 | - |
| Herbasol Destillat Hamamelis | 1,0 | - | 1,0 | - |
| D-Panthenol | 0,7 | 0,525 | 0,6 | 0,525 |
| Hydroglycolic Extract of Ginseng | - | 0,45 | - | 0,45 |
| DSH-C N | 5,0 | 3,0 | - | 3,0 |
| Natriumascorbylphosphat | 1 | - | 1 | - |
| Kombuchka | - | 3 | 3 | 3 |
| Glistin | - | 2 | 1 | 2 |
| Sensiva SC 50 | - | - | - | 0,5 |
| Parfum | 0,10 | 0,10 | 0,10 | 0,10 |
| Aqua | ad 100 | ad 100 | ad 100 | ad 100 |

### 2. Beispielserie: Tagescremes mit Repair-Effekt auf Zellebene

| | 1 | 2 mit "complexion radiance"-Effekt | 3 mit "complexion radiance"-Effekt | 4 |
|---|---|---|---|---|
| Lipoid S75-3 | 0,50 | 0,50 | 0,50 | 0,50 |
| Tocopherylacetat | 0,50 | 0,50 | 0,50 | 0,50 |
| Cutina MD | 1,00 | 1,00 | 1,00 | 1,00 |
| Lanette 22 | 2,00 | 2,00 | 2,00 | 2,00 |
| Baysilone M 350 | 0,50 | 0,50 | 0,50 | 0,50 |
| Propylparaben | 0,20 | 0,20 | 0,20 | 0,20 |
| Dow Corning 9040 | 1,00 | 1,00 | 1,00 | 1,00 |
| Glycerin | 4,50 | 3,00 | 3,00 | 3,00 |
| Hexandiol | 6,00 | 3,00 | | 3,00 |
| Methylparaben | 0,20 | 0,20 | 0,20 | 0,20 |
| Tego Carbomer 140 | 0,30 | 0,30 | 0,30 | 0,30 |
| DS-HCN | 5,00 | 5,00 | 5,00 | 5,00 |
| Propylenglycol | 5,00 | 5,00 | 5,00 | 5,00 |
| Simulgel NS | 1,50 | 1,50 | 1,50 | 1,50 |
| TiO₂ | 0,50 | 0,50 | 0,50 | 0,50 |
| Herbasol Destillat Salbei | | 1,0 | | |
| Herbasol Destillat Hamamelis | | 1,0 | | |
| D-Panthenol | | 0,525 | 0,3 | 0,3 |
| Hydroglycolic Extract of Ginseng | | | 0,45 | 0,45 |
| Natriumascorbylphosphat | 1 | - | 1 | - |
| Kombuchka | - | 3 | 3 | 3 |
| Glistin | - | 2 | 1 | - |
| Ederline L | - | - | - | 2 |
| Matrixyl 3000 | - | - | - | 3 |
| Wasser | ad 100 | ad 100 | ad 100 | ad 100 |

### 3. Beispielserie: Tagescremes mit Repair-Effekt auf Zellebene

| | 1 | 2 mit "complexion radiance"-Effekt | 3 mit "complexion radiance"-Effekt |
|---|---|---|---|
| Sisterna SP30-C | 2,0 | 2,0 | 2,0 |
| Sisterna SP70-C | 0,8 | 0,8 | 0,8 |
| Propylparaben | 0,2 | 0,2 | 0,2 |
| Methylparaben | 0,2 | 0,2 | 0,2 |
| Ethanol | 5,0 | 3,0 | |
| Dry Flo Plus | 1,0 | 1,0 | 1,0 |
| Parfum | 0,3 | 0,3 | 0,3 |
| Cetearyl Alcohol | 1,0 | 1,0 | 1,0 |
| Fucogel 1000 | 2,0 | 1,0 | 0,5 |
| Herbasol Destillat Salbei | 1,0 | 0,5 | 1,0 |
| Herbasol Destillat Hamamelis | 1,0 | 0,5 | |
| D-Panthenol | 0,7 | | |
| Herbasol Destilat weisser Tee | | 0,5 | 0,5 |
| Hydroglycolic Extract of Ginseng | | 0,45 | 0,45 |
| Spirulina Extrakt 3002 | 1,00 | 1,00 | 1,00 |
| DSH-C N | 5,0 | 3,0 | |
| Keltrol SF | 0,2 | | 0,2 |
| Aristoflex AVC | | 0,5 | |
| Hexandiol-1,6 | 5,0 | | |
| Glycerin | 10,0 | 5,0 | 10,0 |
| Dow Corning 200 Fluid | 7,0 | | |
| Dow Corning 245 | | 5,0 | 5,0 |
| Cetiol 868 | 2,0 | | 2,0 |
| Natriumascorbylphosphat | 1 | - | 1 |
| Kombuchka | - | 3 | 3 |
| Glistin | - | 2 | 1 |
| Parfum | 0,3 | 0,3 | 0,3 |
| Wasser | ad 100 | ad 100 | ad 100 |

### 4 Beispielserie: Nachtcremes mit Repair-Effekt auf Zellebene

| | 1 | 2 mit "complexion radiance"-Effekt | 3 mit "complexion radiance"-Effekt |
|---|---|---|---|
| Dow Corning 245 Fluid | 25 | 25 | 25 |
| Abil EM 90 | 2 | 2 | 2 |
| Belsil DM 100 | 2,5 | 2,5 | 2,5 |
| Hydrogenated Castor Oil | 2 | 2,5 | 2 |
| Glycerin | 5 | 5 | 5 |
| NaCl | 2 | 2,1 | 2 |
| Symdiol68 | 0,5 | 0,5 | 0,5 |
| Phenoxyethanol | 0,4 | 0,4 | 0,4 |
| D-Panthenol | 0,45 | 0,45 | 0,45 |
| D,L-alpha-Bisabolol | 0,1 | 0,1 | 0,1 |
| Natriumascorbylphosphat | 1 | - | 1 |
| Kombuchka | - | 3 | 3 |
| Glistin | - | 2 | 1 |
| Parfum | 0,3 | 0,3 | 0,3 |
| Wasser | ad 100 | ad 100 | ad 100 |

### 5. Beispielserie: Tagescremes mit Repair-Effekt auf Zellebene

| | 1 | 2 mit "complexion radiance"-Effekt | 3 mit "complexion radiance"-Effekt |
|---|---|---|---|
| Montanov 202 | 5,0 | 5,0 | 5,0 |
| Cutina MD | 2,0 | 2,0 | 2,0 |
| Cetearyl Alcohol | 1,0 | 1,0 | 1,0 |
| Cetiol B | 9,0 | 9,0 | 9,0 |
| Controx KS | 0,05 | 0,05 | 0,05 |
| Dow Corning 245 | | 7,0 | 3,0 |
| Dow Corning 9040 | 1,0 | 1,0 | |
| Parfum | 0,3 | 0,3 | 0,3 |
| Fucogel 1000 | | 2,0 | |
| Herbasol Destillat Salbei | | | 1,0 |
| Herbasol Destillat Hamamelis | | | 1,0 |
| D-Panthenol | | 0,7 | 0,7 |
| Herbasol Destillat weißer Tee | | 0,5 | |
| Hydroglycolic Extract of Ginseng | | 0,45 | |
| DSH-C N | 5,0 | 5,0 | |
| Keltrol SF | | 0,2 | 0,2 |
| Aristoflex AVC | | | 0,5 |
| Hexandiol-1,6 | 6,0 | 6,0 | |
| Glycerin | 3,0 | 5,0 | 10,0 |
| Dow Corning 200 Fluid | 7,0 | 7,0 | |
| Dow Corning 245 | | 5,0 | 3,0 |
| Dow Corning 9040 | 1,0 | | 1,0 |
| Propylenglycol | 2,0 | 2,0 | |
| Tego Carbomer | 0,3 | 0,3 | 0,3 |
| Phenonip | 0,5 | 0,5 | 0,5 |
| Dry Flo Plus | 1,0 | 1,0 | 1,0 |
| Natriumascorbylphosphat | 1 | - | 1 |
| Kombuchka | - | 3 | 3 |
| Glistin | - | 2 | 1 |
| Wasser | ad 100 | ad 100 | ad 100 |

### 6. Beispielserie: Tagescremes mit Repair-Effekt auf Zellebene

| | 1 | 2 mit "complexion radiance"-Effekt | 3 mit "complexion radiance"-Effekt |
|---|---|---|---|
| Glucamate SSE-20 | 2,0 | 2,0 | 2,0 |
| Sympatens-BS/080 | 2,0 | 2,0 | 2,0 |
| Dry Flo Plus | | 1,0 | 1,0 |
| Parfum | 0,3 | 0,3 | 0,3 |
| Silikonöl 350 | 0,3 | 0,3 | |
| Cutina MD | 2,0 | 2,0 | 2,0 |
| Paraffinöl | 2,0 | 1,0 | |
| Cetearyl Alcohol | 1,0 | 1,5 | 1,25 |
| Capryl-/Caprinsäure-Triglycerid | | 3,5 | 3,0 |
| Herbasol Destillat Salbei | 1,0 | | 1,0 |
| Herbasol Destillat Hamamelis | 1,0 | | 1,0 |
| D-Panthenol | 0,7 | | 1,0 |
| D,I-Bisabolol | 0,1 | 0,1 | |
| Phenoxyethanol | 0,98 | 0,5 | 0,9 |
| Symdiol 68 | | 0,5 | |
| DSH-C N | | 5,0 | |
| Keltrol SF | | | 0,2 |
| Aristoflex AVC | | 0,5 | 0,5 |
| Hexandiol-1,6 | | 5,0 | 5,0 |
| Glycerin | 2,0 | 5,0 | 10,0 |
| Culminal MHPC 100 | 0,1 | 0,1 | 0,1 |
| Propylenglycol | 2,0 | 5,0 | 3,0 |
| Natriumascorbylphosphat | 1 | - | 1 |
| Kombuchka | - | 3 | 3 |
| Glistin | - | 2 | 1 |
| Wasser | ad 100 | ad 100 | ad 100 |

### 2. Reinigungszubereitungen

### 1. Beispielserie: Gesichtswässer (Tonics) mit Repair-Effekt auf Zellebene

| | 1 | 2 mit "complexion radiance"-Effekt | 3 mit "complexion radiance"-Effekt |
|---|---|---|---|
| Dipropylenglycol | 10,00 | 10,00 | 10,00 |
| Chlorhexidindigluconat | 1,00 | 1,00 | 1,00 |
| Synperonic PE L 64 | 3,00 | 3,00 | 3,00 |
| D-Panthenol | 0,50 | 0,50 | 0,50 |
| Hydagen CMF | 3,00 | 3,00 | 3,00 |
| PEG-40 Hydrogenated Castor Oil / Trideceth 9 / Propylene Glycol | 0,50 | 0,50 | 0,50 |
| Natriumascorbylphosphat | 1 | - | 1 |
| Kombuchka | - | 3 | 3 |
| Glistin | - | 2 | 1 |
| Parfume | 0,20 | 0,20 | 0,20 |
| Aqua | ad 100 | ad 100 | ad 100 |

### Liste der verwendeten Rohstoffe

| | INCI | Hersteller/Lieferant |
|---|---|---|
| Abil EM 90 | CETYL PEG/PPG-10/1 DIMETHICONE | Evonik Degussa (Goldschmidt) |
| Aristoflex AVC | Ammonium Acryloyldimethyltaurate/VP Copolymer, t-Butyl Alcohol | Clariant |
| Baysilon M 350 | Dimethicone (350 cSt) | GE Bayer Silicones |
| Belsil DM 100 | Dimethicone (100 cSt) | Wacker |
| Cetiol^{®} 868 | Ethylhexyl Stearate | Cognis |
| Cetiol^{®} B | Dibutyl Adipate | Cognis |
| Controx KS | Tocopherol, Hydrogenated Palm Glycerides Citrate | Cognis |
| Culminal MHPC 100 | Hydroxypropyl Methyl Cellulose | Hercules |
| Cutina MD | Glyceryl Stearate | Cognis |
| Dow Corning 200 (0,65 cSt) | Dimethicone | Dow Corning |
| Dow Corning 9040 | Cyclomethicone/ Dimethicone Crosspolymer (87:13) | Dow Corning |
| Dow Corning^{®}200 Fluid, 5 cSt. | Dimethicone | Dow Corning |
| Dry Flo Plus | Aluminium Starch Octenylsuccinate | National Starch |
| DSH CN | Water, Dimethylsilanol Hyaluronate | Exsymol |
| DURO-TAK^{®} 387-2516 | Polyacrylate, Ethylcellulose, Polyvinylpyrrolidon | National Starch and Chemical |
| Ederline L | Hexyldecanol, Pyrus Malus (Apple) Fruit Extract | Seporga |
| Fucogel 1000 | Aqua (Water), Biosaccharide Gum-1 | Solabia |
| Gingkoselect Phytosome | 0,6 - 1,2 Gew.-% Ginsenoside-Aktivsubstanz, Liposomen-verkapselt und unverkapselt | Indena SpA |
| Glistin | AQUA (WATER), GLUTAMYLAMIDOETHYL INDOLE (0,9-1,1 Gew.-% Aktivsubstanz), Methylparaben (0,12 - 0,15 Gew.-%) | Exsymol |
| Glucamate^{®} SSE-20 | PEG-20 Methyl Glucose Sesquistearate | AMERCHOL |
| Hydagen CMF | Chitosan Glycolate | Cognis |
| Hydrovance | Bis-N, N'-(2-Hydroxyethyl)harnstoff (ca. 50 Gew.-%), Wasser, Harnstoff, Ammoniumlactat | National Starch |
| Keltrol SF | Xanthan Gum | Kelco |
| Kombuchka | Aqua (Water), Saccharomyces/Xylinum/ Black Tea Ferment, Glycerin, Hydroxyethylcellulose | Sederma |
| Lanette^{®} 22 | Behenyl Alcohol | Cognis |
| Lipoid S 75-3 | Aqua, Lecithine | Lipoid GmbH |
| Matrixyl | Aqua, Palmitoyl Pentapeptide-3 | Sederma |
| Matrixyl 3000 | Glycerin, Aqua, Butylene Glycol, Carbomer, Polysorbate 20, Palmitoyl Oligopeptide, Palmitoyl Tetrapeptide-1 | Sederma |
| Montanov 202 | Arachidyl Alcohol, Behenyl Alcohol, Arachidyl Glucoside | Seppic |
| Montanov L | C14-22 Alcohols/C12-20 Alkyl Glucoside | Seppic |
| Myritol 318 | Caprylic/Capric Triglyceride | Cognis |
| Novata AB | Cocoglycerides | Cognis |
| Phenonip^{®} | Phenoxyethanol, Methylparaben, Ethylparaben, Propylparaben, Butylparaben, ca. 28 % Aktivsubstanz | NIPA |
| Sensiva^{®} SC 50 | 2-Ethylhexylglycerinether | Schülke & Mayr |
| Simulgel NS | Hydroxyethyl Acrylate / Sodium Acryloyldimethyl Taurate Copolymer / Squalane / Polysorbate 60 | Seppic |
| Sisterna SP 30-C | Sucrose distearate | Sisterna B.V. |
| Sisterna SP 70-C | Sucrose stearate | Sisterna B.V. |
| Stenol 16/18 | Cetearylalkohol | Cognis |
| Symdiol 68 | 1,2-Octandiol, 1,2-Hexandiol | Symrise |
| Sympatens-BS/080 | PEG-8 stearate | Dr. W. Kolb |
| Synperonic PE L 64 | Poloxamer-184 | Uniqema |

## Patentansprüche

1. Verwendung von mindestens zwei verschiedenen Wirkstoffen, ausgewählt aus Glutamylamidoethylindol, Kombucha, Hypotaurin, Ascorbinsäure und deren Salzen, Ascorbinsäureestern und Ascorbinsäureethern, zur Herstellung einer kosmetischen oder pharmazeutischen Zusammensetzung zur Verbesserung der "complexion radiance".

2. Verwendung von mindestens einem Wirkstoff, ausgewählt aus Glutamylamidoethylindol, Kombucha, Hypotaurin, Ascorbinsäure und deren Salzen, Ascorbinsäureestern, Ascorbinsäureethern und Mischungen dieser Wirkstoffe, zur Herstellung einer kosmetischen oder pharmazeutischen Zusammensetzung zur Reduzierung der zellulären Proteinoxidation in Keratinozyten.

3. Verwendung von mindestens einem Wirkstoff, ausgewählt aus Glutamylamidoethylindol, Kombucha, Hypotaurin, Ascorbinsäure und deren Salzen, Ascorbinsäureestern, Ascorbinsäureethern und Mischungen dieser Wirkstoffe, zur Herstellung einer kosmetischen oder pharmazeutischen Zusammensetzung zur Steigerung der Proteasomen-Aktivität in Keratinozyten bzw. zur Beibehaltung der Proteasomen-Aktivität in Keratinozyten unter UV-Einfluss.

4. Verwendung von Kombucha zur Herstellung einer kosmetischen oder pharmazeutischen Zusammensetzung zur Steigerung der Lamininsynthese in der extrazellulären Matrix epidermaler Keratinozyten.

5. Verwendung gemäß einem der Ansprüche 1 - 3, **dadurch gekennzeichnet, dass** eine Wirkstoffkombination aus Glutamylamidoethylindol und Kombucha verwendet wird.

6. Verwendung gemäß einem der vorstehenden Ansprüche, **dadurch gekennzeichnet, dass** das Kombucha aus Tee von fermentierten (Schwarztee, Oolong-Tee, Gelber Tee, Pu-Erh-Tee) oder unfermentierten Blättern (Grüntee) von Thea sinensis (Camellia sinensis) oder Thea assamica gewonnen ist.

7. Verwendung gemäß einem der vorstehenden Ansprüche, **dadurch gekennzeichnet, dass** das Kombucha durch Fermentation von gezuckertem Tee mit den symbiotischen Mikroorganismen Saccharomyces und Acetobacter xylinum gewonnen ist.

8. Verwendung gemäß einem der vorstehenden Ansprüche, **dadurch gekennzeichnet, dass** 0,0001 - 0,1 Gew.-%, bevorzugt 0,001 - 0,05 Gew.-%, besonders bevorzugt 0,001 - 0,02 Gew.-%, jeweils bezogen auf das Gesamtgewicht der Zusammensetzung, an Glutamylamidoethylindol enthalten ist.

9. Verwendung gemäß einem der vorstehenden Ansprüche, **dadurch gekennzeichnet, dass** 0,0001 - 10 Gew.-%, bevorzugt 0,01 - 5 Gew.-%, besonders bevorzugt 0,1 - 3 Gew.-%, jeweils bezogen auf das Gesamtgewicht der Zusammensetzung, an Kombucha enthalten ist.

10. Verwendung gemäß einem der Ansprüche 1 - 3, **dadurch gekennzeichnet, dass** das Ascorbinsäurederivat ausgewählt ist aus den Estern der Ascorbinsäure mit mindestens einer organischen und/oder anorganischen Säure.

11. Verwendung gemäß einem der Ansprüche 1 - 3, **dadurch gekennzeichnet, dass** das Ascorbinsäurederivat ausgewählt ist aus den Ethern der Ascorbinsäure mit mindestens einer organischen Polyhydroxyverbindung, insbesondere mit mindestens einem Saccharid.

12. Verwendung gemäß einem der vorstehenden Ansprüche, **dadurch gekennzeichnet, dass** weiterhin mindestens ein Wirkstoff enthalten ist, der ausgewählt ist aus
- Monomeren und Oligomeren von Aminosäuren, N-C₂-C₂₄-Acylaminosäuren, den Estern und/oder den physiologisch verträglichen Metallsalzen dieser Substanzen,
- DNA-Reparaturenzymen,
- DNA- oder RNA-Oligonucleotiden,
- Vitaminen, Provitaminen und Vitaminvorstufen der Gruppen A, B, E, H und K und den Estern der vorgenannten Substanzen,
- α-Hydroxycarbonsäuren, α-Ketocarbonsäuren, β-Hydroxycarbonsäuren und deren Ester-, Lacton- und/oder Salzform,
- Flavonoiden und Flavonoid-reichen Pflanzenextrakten,
- Ubichinon und Ubichinol sowie deren Derivaten,
- Silymarin,
- Ectoin,
- anorganischen und organischen UV-Filtersubstanzen,
- selbstbräunenden,
- hautberuhigenden,
- feuchtigkeitsspendenden und
- sebumregulierenden Wirkstoffen sowie
- Mischungen dieser Wirkstoffe.
